# EUROPEAN PATENT SPECIFICATION

(11) **EP 1 588 755 B1**
(45) Date of publication and mention of the grant of the patent: **17.06.2009**
(21) Application number: 03775908.1
(22) Date of filing: 26.11.2003
(51) Int. Cl.: G01N 33/543, B01D 57/02, B03C 5/00, G01N 33/53, C12N 15/00

(54) **DEVICE AND METHOD FOR PROTEIN DETECTION**
VORRICHTUNG UND METHODE ZUR PROTEINDETEKTION
DISPOSITIF ET PROCEDE POUR LA DETECTION DE PROTEINES

(30) Priority: 28.03.2003 JP 2003092007
(43) Date of publication of application: 26.10.2005
(73) Proprietor: FUJITSU LIMITED, Kawasaki-shi, Kanagawa 211-8588 (JP)
(72) Inventor: FUJIHARA, Tsuyoshi, Kawasaki, Kanagawa 211-8588 (JP); FUJITA, Shozo, Kawasaki, Kanagawa 211-8588 (JP); TAKEISHI, Shunsaku, Kawasaki, Kanagawa 211-8588 (JP); ARINAGA, Kenji, Kawasaki, Kanagawa 211-8588 (JP); YAMAGUCHI, Yoshitaka, Kawasaki, Kanagawa 211-8588 (JP); USUKI,Tatsuya, Kawasaki, Kanagawa 211-8588 (JP)
(74) Representative: Brown, David Leslie
(86) International application number: PCT/JP2003/015099
(87) International publication number: WO 2004/087303

(56) References cited:
- WO-A-97/12030
- WO-A1-97/12030
- WO-A1-99/63885
- US-A- 5 434 049
- US-A- 5 434 049
- BOON ELIZABETH M ET AL: "An electrical probe of protein-DNA interactions on DNA-modified surfaces" NATURE BIOTECHNOLOGY, vol. 20, no. 3, March 2002 (2002-03), pages 282-286, XP002980572 ISSN: 1087-0156
- TAKEISHI SHUNSAKU ET AL: "Observation of electrostatically released DNA from gold electrodes with controlled threshold voltages." THE JOURNAL OF CHEMICAL PHYSICS 22 MAR 2004, vol. 120, no. 12, 22 March 2004 (2004-03-22), pages 5501-5504, XP008077654 ISSN: 0021-9606

## Description

### Technical Field

A target detecting device is provided capable of efficiently detecting a variety of targets such as proteins without labeling typically with fluorescence and to a target capturer that can be suitably used therein.

A device and method are provided for molecular adsorption or desorption which are capable of efficiently and reliably adsorbing and/or desorbing one or more useful molecules such as DNAs with different timings arbitrarily, are capable of, for example, being down-sized, formed into chips and/or integrated and show high performance.

A device and method are provided for protein detection which are capable of detecting and quantitatively determining one or more protein without labeling.

### Background Art

The human genome project has been considered to bring a large change in paradigm to scientific technology and industries involving life sciences. For example, diabetes mellitus has been classified based on symptom of high blood glucose level and, with respect to the cause of onset, has been classified, based on a level of capacity of producing insulin in a patient's body, into type I and type II. However, more detailed classification by more detailed diagnosis can be achieved by using the information on human genome sequence presented by the human genome project, which in turn achieves more appropriate treatment. More specifically, the human genome project reveals all the data of amino-acid sequence of proteins, such as enzymes and receptors, pertaining to the control typically of the detection, synthesis and/or decomposition of blood glucose and insulin, and a DNA sequence of genes pertaining to the control of the abundances of such proteins. Using such data, diabetes mellitus can be classified into subtypes, depending on which protein or proteins of the above-listed proteins causing abnormal control of the blood level, and, accordingly, it must become possible to carry out an appropriate treatment. Thus, the conditions of a series of proteins functionally involved in a specific disease can be grasped by using the information on the human genome sequence, which enables a more appropriate diagnosis and/or treatment of the disease.

To make this possible, a technique enabling simple measurement of the amounts of a series of proteins, which are functionally related to a specific disease, is needed. However, such a technique is being developed as a technique of analyzing proteome. As a currently established method, there is known a method in which measurement is carried out by using two-dimensional electrophoresis in combination with mass spectrometric system. This technique, however, cannot satisfactorily and rapidly grasp a patient's symptoms at a clinical site, such as a laboratory or at the bedside in a hospital.

Alternatively, certain analysis techniques use a DNA chip. Such a DNA chip is designed to be adapted for the determination of a DNA in a sample to be detected by previously introducing thereinto a fluorescent pigment during the amplification (increment) thereof by a PCR (polymerase chain reaction), and determining the amount of DNA bound to complementary DNA strands arranged on a chip in the form of array by the intensity of fluorescence. These techniques using a DNA chip, however, are not suitable for the detection of proteins. Namely, proteins cannot be processed by what corresponds to amplification in the PCR reaction. In addition, when a sample contains a mixture of plural kinds of proteins, a fluorescent label cannot be uniformly introduced into them because the respective proteins have different reactivities with the fluorescent dye.

Separately, there is disclosed a technique in which a DNA is combined to a gold electrode with the interposition of a thiol group, and a negative potential is applied to the gold electrode to thereby allow the substrate to desorb the DNA (for example, Non-patent Document 1). This document, however, fails to disclose how the technique to be applied.
Non-patent Document 1
J. Wang et al., Langmuir,15, 6541-6545 (1999)

The present invention has been accomplished to solve the problems in conventional technologies and to achieve the following objects. Specifically, an object of the present invention is to provide a target detecting device capable of efficiently detecting a variety of targets such as proteins without labeling typically with fluorescence, and to provide a target capturer that can be suitably used therein.

Recent years have seen significant and rapid advances in the area of biotechnology industry and development of novel techniques in diagnosis/ treatment of diseases.

For example, Patent Document 1 discloses a DNA chip for determining the amount of a DNA to be determined based on the intensity of fluorescence, the DNA being bound to complementary DNA strands arranged in an array. The DNA chip, however, is prepared by applying and immobilizing DNA molecules to specific positions typically using a spotter, requires complicated procedures, invites high cost and is not suitable for mass production. This problem becomes more significant with increasing types of the DNA molecules. A demand has been made to develop a technique that is capable of easily and conveniently immobilizing many types of DNA molecules to a DNA chip.

If a DNA chip after reacting with a DNA to be detected can be retained, each of DNAs to be tested can be sampled from the chip and subjected to amplification and then to diagnosis and/ or analysis. In addition or alternatively, if the DNA chip is so designed as to desorb the DNA molecule, the DNA can be used repetitively and is very convenient Demands have been made to develop these techniques for further advance in research and development in the area of biotechnologies.

Separately, demands have been made typically in the area of diagnosis/treatment of diseases to develop techniques that can quantitatively determine not only DNAs but also various substances or molecules at desired timings or can supply such substances or molecules to an arbitrary subject. These techniques must control the migration of the substance or molecule. The control, however, is very difficult. Namely, conventional devices for diagnosing or analyzing, such as DNA chips, cannot arbitrarily control the movement of the subject to be diagnosed or analyzed, such as a DNA molecule, cannot be arbitrarily control the migration of, for example, the DNA molecule. In addition, the reaction system cannot be significantly retained after allowing the subject to be diagnosed or analyzed to react with the diagnosing/analyzing device.

Thus, there has been provided no technique which is capable of efficiently and reliably adsorbing and/ or desorbing one or more useful molecules such as DNAs with different timings arbitrarily, is capable of, for example, being down-sized, formed into chips and/or integrated and show high performance and is suitable typically for gene therapy, or diagnosis and/or analysis.
Patent Document 1
Japanese Patent Application Laid-Open (JP-A) No. 11-512605

Under these circumstances, the present invention has been accomplished to meet the demands, to solve the problems in conventional technologies and to achieve the following objects. Specifically, an object of the present invention is to provide a device for molecular adsorption or desorption which is capable of efficiently and reliably adsorbing and/or desorbing one or more useful substances or molecules, such as DNAs with different timings arbitrarily, is capable of down-sized, formed into chips or integrated, is suitable typically for gene therapy, diagnosis and/or analysis and is safe. Another object of the present invention is to provide a method for molecular adsorption or desorption which is capable of efficiently and reliably adsorbing or desorbing one or more useful substances or molecules, such as DNAs, with different timings arbitrarily, is suitable typically for diagnosis and/or analysis and is safe.

The human genome project, developed in 1990s, was an attempt for some countries to share decoding of all of the human genetic code. It has been announced that a draft was finished in the summer of 2000. It is expected that, as functional genome science and structural genome science progress after this, it will be revealed what function each of the decoded human genome sequence data pertains to.

The human genome project has brought a large change in paradigm to scientific technology and industries involving life sciences. For example, diabetes mellitus has been classified based on symptom of high blood sugar level and, with respect to the cause of onset, has been classified, based on a level of capacity of producing insulin in a patient's body, into type I and type II. The human genome project presents all the data of amino-acid sequence of proteins, such as enzymes and receptors, pertaining to the control of the detection of blood glucose and insulin, or synthesis, decomposition and the like of insulin, and a DNA sequence of genes pertaining to the control of the amounts of such proteins exists.

Using such data, diabetes mellitus, a phenomenon of the control of blood glucose level being not functioned, can be classified into subtypes, depending on what proteins pertaining to the process of, for example, the synthesis and decomposition of insulin are upset, and, accordingly, it must become possible to carry out an appropriate diagnosis and treatment.

Particularly, development of new drugs based on the genome data, in which drugs are developed for particular proteins based on the human genome sequence, is being energetically promoted by the pharmaceutical industry, and it is expected that the mitigation and cure of a symptom will be effected by understanding the conditions of a sequence of proteins which are functionally related to each other for the symptom and by administering a genetically developed drug.

To make this possible, a technique enabling simple measurement of the amounts of a sequence of proteins, which are functionally related to each other, is needed. However, such a technique is being developed as a technique of analyzing proteome.

As a currently established method, there is known a method in which measurement is carried out by combining two-dimensional electrophoresis and mass spectrometric analysis, which requires a relatively large-scale apparatus. To determine a patient's symptoms at a clinical site, such as a laboratory or at the bedside in a hospital, the development of a simple, novel technique is needed.

A so-called DNA chip is designed to be adapted for the determination of a DNA in a sample to be detected by previously introducing thereinto a fluorescent dye group during the amplification (increment) thereof by a PCR (polymerase chain reaction), and determining the amount of DNA bound to complementary DNA strands arranged on a chip in an array by the intensity of fluorescence.

In contrast, proteins cannot be processed by what corresponds to amplification by the PCR reaction in the case of DNA. In addition, when a sample contains a mixture of plural kinds of proteins, a fluorescent label cannot be uniformly introduced into them because the individual molecules have different reactivities with the fluorescent dye.

Accordingly, yet another object of the present invention is to solve the problems and to provide a protein detecting device and method for easily and conveniently detecting and determining one or more proteins.

### Disclosure of Invention

The device for protein detection of the present invention comprises a binding section capable of binding specifically to a protein to be detected; a sensing section for detecting the binding of the protein to the binding section, the sensing section comprising a nucleotide strand and a fluorescent dye group; a first electrode serving to immobilize the sensing section; a second electrode; a control section for controlling the conformation of the sensing section, the control section including the first electrode and the second electrode; and a detecting section or detecting emission or quenching of light by the sensing section wherein the binding section comprises at least one selected from the group consisting of an IgG antibody, a Fab fragment of an IgG antibody, and a fragment derived from a Fab fragment of an IgG antibody, and
wherein the binding section is attached to the sensing section. The device is so configured as to apply an electric field between the said first and the second electrodes being constant or varying with time.

The method for protein detection of the present invention comprising the steps of arranging a protein detecting unit on or above a first electrode, the protein detecting unit comprising a binding section capable of binding specifically to a protein to be detected and a sensing section for detecting the binding of the protein to the binding section, the sensing section comprising a nucleotide strand and a fluorescent dye group; immersing the electrode in a sample mixture containing a protein; applying an electric field to between the first electrode and a second electrode, the electric field having a constant or time-variant potential difference, the second electrode being placed in the sample mixture; and detecting at least one of emission and quenching of light by the sensing section.

The term "nucleotide" herein means one selected from the group consisting of mononucleotide, oligonucleotides and polynucleotides, or a mixture of these nucleotides.

### Brief Description of the Drawings

Fig.1 is a schematic explanatory diagram showing an example of the target capturer of the present invention.
Fig. 2 is a schematic explanatory diagram showing light emission of a light emitting section of the target capturer shown in Fig. 1.
Fig. 3 is a schematic explanatory diagram showing a target capturer immobilized to an electrically conductive substrate.
Fig. 4 is a schematic explanatory diagram showing release of the target capturer by the action of means for releasing the target capturer.
Fig. 5 is a schematic explanatory diagram showing an example of a target capturer immobilized to an electrically conductive substrate so as not to be released.
Fig. 6 is a schematic explanatory diagram showing an example of detection of the presence of a target using the target detecting device of the present invention.
Fig. 7 is a graph showing a result of the detection by the target detecting device of the present invention when a target protein is absent.
Fig. 8 is a graph showing a result of the detection by the target detecting device of the present invention when a target protein is present.
Fig. 9 is a graph showing a result of the detection of the presence or absence of a target avidin by the light detecting means of target detecting device of the present invention.
Figs. 10A and 10B are each a schematic explanatory diagram showing an example of adsorption/desorption electrodes immobilized to a substrate.
Fig. 11 is a schematic explanatory diagram showing an example of two or more adsorption/ desorption electrodes, a counter electrode and reference electrodes immobilized to a substrate.
Fig. 12 is a schematic explanatory diagram showing an example of an integrated device for molecular adsorption or desorption including the substrates shown in Fig. 11.
Fig. 13 is a schematic explanatory diagram showing the principle of adsorption of DNA molecules by an adsorption/ desorption electrode.
Fig. 14 is a schematic explanatory diagram corresponding to Fig. 13, except with a reference electrode.
Figs. 15A and 15B are each a schematic explanatory diagram illustrating the principle of desorption of DNA molecules from an adsorption/ desorption electrode.
Fig. 16 is a schematic explanatory diagram showing an example of molecules adsorbed by an adsorption/desorption electrodes.
Fig. 17 is a schematic explanatory diagram showing an example of desorption of molecules from an adsorption/ desorption electrode.
Fig. 18 is a schematic explanatory diagram showing an example of molecules having a light emitting section.
Fig. 19 is a schematic explanatory diagram showing an example of light emission of the light emitting section shown in Fig. 18.
Fig. 20 is a first schematic diagram explaining detection of the presence of a target using molecules having a light emitting section.
Fig. 21 is a second schematic diagram explaining detection of the presence of a target using molecules having a light emitting section.
Fig. 22 is a graph showing test data for determining properties of an adsorption/ desorption electrode (gold electrode) by cyclic voltammetry.
Fig. 23 is a graph of test data showing desorption of a molecule having a light emitting section from an adsorption/ desorption electrode using molecules having a light emitting section.
Fig. 24 is a cross sectional view showing an example of the device for protein detection according to the present invention.
Fig. 25 illustrates emission or quenching of fluorescence of protein detecting unit on an electrode as a result of the application or removal of an electric field.
Fig. 26 is a diagram showing a change of emission intensity with time when a protein is not bound.
Fig. 27 is a diagram showing a change of emission intensity with time when a protein is bound.
Fig. 28 is a diagram showing a change of emission intensity with time upon application of a voltage having a pulse waveform when a protein is not bound.
Fig. 29 is a diagram showing a change of emission intensity with time upon application of a voltage having a pulse waveform when a protein is bound.
Fig. 30 is a schematic diagram showing a plurality of circular electrodes separately arranged on an electrode supporting section.

### Best Mode for Carrying Out the Invention

### (Target Capturer) .

The target capturer of the present invention comprises an interacting section, a target capturing section and a light emitting section and may further comprise one or more other sections or units appropriately selected according to necessity.

### -Interacting Section-

The interacting section is not specifically limited and can be appropriately selected according to the purpose, as long as it at least partially contains a region interactive with an electrically conductive member.

The interaction is not specifically limited, can be appropriately selected according to the purpose and includes, for example, electrical actions such as electrical coupling; chemical actions such as chemical binding; and physical actions such as adsorption.

The shape of the interacting section is not specifically limited, can be appropriately selected according to the purpose and is preferably, for example, a linear shape.

Preferred examples of the interacting section are ionic polymers, since they are electrically interactive (e.g., binding or coupling) with the electrically conductive member.

The ionic polymers are preferably selected from cationic polymers and anionic polymers.

Suitable examples of the cationic polymers (positively charged ionic polymers) are guanidine DNA and polyamines.

Suitable examples of the anionic polymers (negatively charged ionic polymers) are polynucleotides and poly(phosphoric acid)s. These substances are preferred, since they have negative charges dispersed at constant intervals in the molecule and serve to easily control the interaction such as coupling with the electrically conductive member.

The polynucleotide, if used, is preferably selected from DNAs and RNAs. The DNAs and RNAs may each be single-stranded or double-stranded.

A method for preparing the polynucleotide is not specifically limited, can be appropriately selected from known methods according to the purpose and includes, for example, a method using a DNA synthesizer (DNA automatic synthesis system), and a method in which an oligonucleotide sequence previously prepared is subjected to annealing with arrayed monomer blocks, and a DNA ligase or an RNA ligase is allowed to react to thereby bind them.

The length of the polynucleotide is not specifically limited, can be appropriately selected according to the purpose and is preferably at least six bases. A peak voltage required for allowing the electrically conductive member to desorb the polynucleotide generally deceases with a decreasing length of the polynucleotide.

The interacting section may comprise portions having different interactivities, such as associative strength, with the electrically conductive member. Thus, the polynucleotide, for example if used as the interacting section may have introduced groups such as (CH₂)₃ SS (CH₂)₃OH group at its three prime-end.

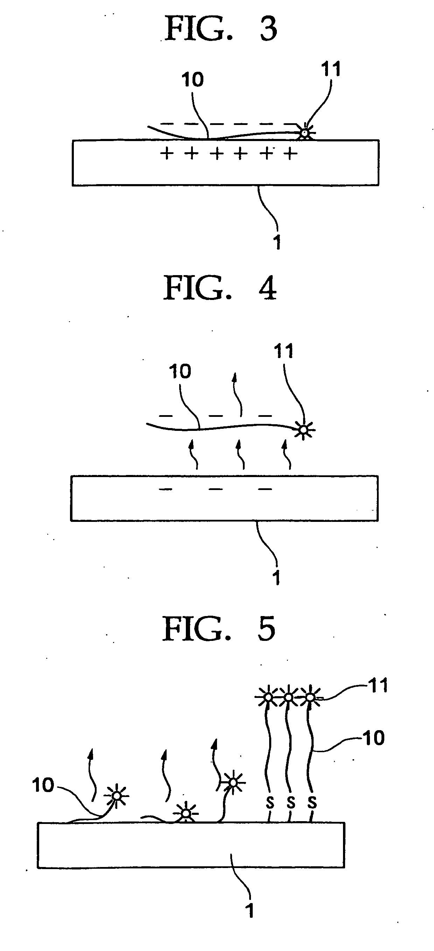

In this case, the SS moiety of the group is rigidly bound to the electrically conductive member, such as a metal electrode. The target capturer rigidly bound to the electrically conductive member is not desorbed therefrom when a low voltage is applied to the electrically conductive member (Fig. 5). Thus, the target capturer can be one capable of being desorbed from the electrically conductive member at a low voltage or be one unable to be desorbed from the electrically conductive member unless at a high voltage. The former can be advantageously applied to a target detecting device capable of detecting the presence of the target at low cost. The latter can be applied to a target detecting device that is resistant to, for example, fluctuations in environmental conditions.

The number of the interacting section per one molecule of the target capturer is not specifically limited, can be appropriately selected according to the purpose, is at least one and may be two or more.

The electrically conductive member is not specifically limited, can be appropriately selected according to the purpose, as long as it has electrical conductivity, and includes, for example, known electrode substrates, of which a metal electrode is preferable.

Examples of the metal electrode substrate are gold electrodes and copper electrodes. The surfaces of these are preferably burnished or polished.

One or more reference electrodes are preferably arranged with respect to the electrically conductive member when an electric field is applied to the electrically conductive member. The reference electrode is not specifically limited and can be appropriately selected according to the purpose from among known electrodes.

When the interacting section of the target capturer comprises an ionic polymer, an electric field opposite to the electrostatic potential of the ionic polymer is applied to the electrically conductive member in order to maintain the interaction, such as binding or coupling, of the ionic polymer with the electrically conductive member. In contrast, an electric field identical to the electrostatic potential of the ionic polymer is applied to the electrically conductive member in order to cease the interaction, such as binding or coupling, between the ionic polymer and the electrically conductive member to thereby allow the electrically conductive member to desorb the ionic polymer. The electric field is not specifically limited, can be appropriately selected according to the purpose and may be a direct-current electric field or an alternating-current electric field.

A standard electrode may be arranged when the reference electrode is used. This configuration can advantageously easily control the electric potential between the electrically conductive member and the reference electrode.

### -Target Capturing Section-

The target capturing section is not specifically limited and can be appropriately selected according to the purpose, as long as it is capable of capturing the target. The aspect of the capture is not specifically limited and includes, for example, adsorption; and chemical binding such as covalent binding, ionic binding, coordinate binding, hydrogen binding and intermolecular force.

Suitable examples of the target capturing section are antibodies, antigens, enzymes and coenzymes with respect to the target. The target capturing section can be selected according to the type of the target. When the target is, for example, an antigen, an antibody against the antigen can be selected as the target capturing section. When the target is an antibody, an antigen against the antibody can be selected as the target capturing section. When the target is an enzyme such as avidin, a coenzyme to the enzyme, such as biotin, can be selected as the target capturing section. When the target is a coenzyme such as biotin, an enzyme to the coenzyme, such as avidin can be selected as the target capturing section.

The number of the target capturing sections per one molecule of the target capturer is not specifically limited, can be appropriately set according to the purpose, is at least one and may be two or more.

The position of the target capturing section in the target capturer is not specifically limited and can be appropriately set according to the purpose. When the interacting section is linear, the target capturing section may be arranged at the end or terminal of the interacting section. When the interacting section is a polynucleotide, the position may be three prime-end and/ or five prime-end.

The target is not specifically limited, can be appropriately selected according to the purpose and includes, for example, organic molecules.

Examples of the organic molecules are proteins, lipoproteins, glycoproteins, polypeptides, lipids, polysaccharides, lipopolysaccharides, nucleic acids and medicaments or drugs. Among them, proteins, plasma proteins, tumor markers, apoproteins, viruses, autoantibodies, coagulation and fibrinogenolysis factors, hormones, medicaments or drugs in the blood, HLA antigens and nucleic acids are preferred, of which proteins are more preferred.

Examples of the proteins are enzymes such as avidin.

Examples of the plasma proteins are immunoglobulins (IgG, IgA, IgM, IgD and IgE)m components of complement (C3, C4, C5 and Clq), CRP, α₁ -antitrypsin, α₁-microglobulin, β₂-microglobulin, haptoglobin, transferrin, ceruloplasmin and ferritin.

Examples of the tumor markers are α-fetoprotein (AFP), carcinoembryonic antigen (CEA), CA 19-9, CA 125, CA 15-3, SCC antigen, prostatic acid phosphatase (PAP), PIVKA-II, γ-seminoprotein, TPA, elastase I, neuron-specific enolase (NSE) and immunosuppressive acidic protein (IAP).

Examples of the apoproteins are Apo A-I, Apo A-II, Apo B, Apo C-II, Apo C-III and Apo E.

Examples of the viruses are hepatitis B virus (HBV), hepatitis C virus (HBC), HTLV-I and HIV. Examples of infective diseases other than viruses are antistreptolysin O (ASO), toxoplasma, mycoplasma and sexually transmitted diseases (STDs).

Examples of the autoantibodies are anti-microsome antibodies, anti-thyroglobulin antibodies, antinuclear antibodies, rheumatoid factors, antimitochondrial antibodies and antimyelin antibodies.

Examples of the congealing fibrinogenolysis factors are fibrinogen, fibrin decomposition products (FDPs), plasminogen, α₂ -plasmin inhibitor, antithrombin III, β-thromboglobulin, Factor VIII, protein C and protein S.

Examples of the hormones are pituitary hormones such as LH, FSH, GH, ACTH, TSH and prolactin; thyroid hormones such as T₃ , T₄ and thyroglobulin; calcitonin; parathormone (PTH); adrenal cortical hormones such as aldosterone and cortisol; gonadal hormones such as hCG, oestrogen, testosterone and hPL; hormones of the pancreas and gastrointestinal tract, such as insulin, C-peptide, glucagon and gastrin; and other hormones such as renin, angiotensin I, angiotensin II, enkephalin and erythropoietin.

Examples of the medicaments or drugs in the blood are antiepileptic agents such as carbamazepine, primidone and valproic acid; agents for cardiovascular diseases, such as digoxin, quinidine, digitoxin and theophylline; and antibiotics such as gentamicin, kanamycin and streptomycin.

Examples of the nucleic acids are cancer-associated genes, genes relating to hereditary diseases, viral genes, bacterial genes, and genes showing polymorphism and called as disease risk factors.

Examples of the cancer-associated genes are k-ras gene, N-ras gene, p53 gene, BRCA1 gene, BRCA2 gene, src gene, ros gene and APC gene.

Examples of the genes relating to hereditary diseases are genes relating to a variety of congenital metabolic dieseases, such as phenylketonuria, alcaptonuria, cystinuria, Huntington chorea, Down syndrome, Duchenne muscular dystrophy and haemophilia.

The viral genes and the bacterial genes include, for example, genes of hepatitis C virus, hepatitis B virus, influenzae virus, measles virus, HIV virus, mycoplasma, rickettsia, streptococci and Salmonella species.

The genes showing polymorphism include genes having different base sequences from individual to individual and being not always directly related to causes of diseases, for example, PS1 (presenilin-1) gene, PS2 (presenilin-2) gene, APP (amyloid beta precursor protein) gene, lipoprotein gene, genes relating to HLA (human leukocyte antigen) or blood typing, and genes believed to be involved in the onset of, for example, hypertension or diabetes.

Examples of a specimen (sample) containing the target are pathogens such as bacteria and viruses; the blood, saliva, pieces of tissues and other substances separated from a living body; excreta such as urine and feces; fetal cells in the amniotic fluid, and part of cleaved ovum in vitro for prenatal diagnosis. Each of these specimens (samples) may be subjected, as intact or after concentrating as a sediment typically by centrifugal separation, to treatment for cell destruction, such as enzymatic treatment, thermal treatment, treatment with a surfactant, supersonic treatment, and combinations of these treatments.

### -Light Emitting Section-

The light emitting section is not specifically limited and can be appropriately selected according to the purpose, as long as it is capable of emitting light upon irradiation with light when the region in the interaction section is not interacting with the electrically conductive member. Suitable examples of the light emitting section are fluorescent dyes, metals and semiconductive nanospheres.

The fluorescent dyes are typically preferred as the light emitting section. When the electrically conductive member is a metal such as a metal electrode, such a fluorescent dye does not emit light even upon irradiation with radiation having such a wavelength that the fluorescent dye can absorb when the fluorescent dye is interacting with the metal (for example, the fluorescent dye is positioned in the proximity to the metal). On the other hand, the fluorescent dye can emit light by the action of light energy upon irradiation with radiation having such a wavelength that the fluorescent dye can absorb, when the dye is not interacting with the metal (for example, the fluorescent dye is positioned away from the metal).

The fluorescent dye is not specifically limited and can be appropriately selected according to the purpose from among known fluorescent dyes. Suitable examples thereof are a compound represented by following Structural Formula 1:

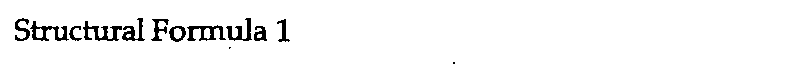

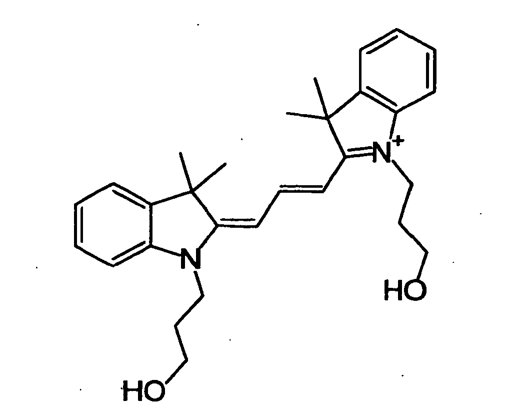

The number of the light emitting sections per one molecule of the target capturer is not specifically limited, can be appropriately selected according to the purpose is at least one and may be two or more.

The position of the light emitting section in the target capturer is not specifically limited and can be appropriately selected according to the purpose. When the interacting section is linear, the target capturing section may be arranged at the end or terminal of the interacting section. When the interacting section is a polynucleotide, the position may be three prime-end and/ or five prime-end.

When the interacting section is the polynucleotide, the target capturer of the present invention can be prepared, for example, by a method of elongating the polynucleotide strand of an oligodeoxyribonucleotide (oligo-DNA) that is modified by a molecule serving as the target capturing section at the five prime-end, using a primer and a DNA polymerase and allowing a molecule serving as the light emitting section to bind with the molecular chain.

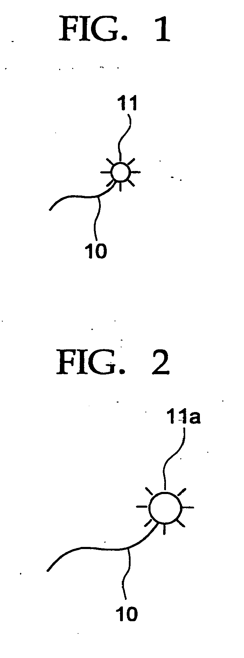

The target capturer will be illustrated with reference to the drawings. Figs. 1 and 2 are each a schematic explanatory diagram showing the target capturer. With reference to Figs. 1 and 2, the target capturer comprises a linear or filamentous interacting section 10, such as a polynucleotide, a light emitting section 11, such as a fluorescent dye, bound to one end of the interacting section 10, and a target capturing section (not shown), such as an antibody, bound to the other end of the interacting section 10. The target capturing section may be arranged at the same end as the light emitting section 11 or in a side chain of the interacting section 10. In Fig.1, the interacting section 10 of the target capturer is interacting with an electrically conductive member, such as a metal electrode , and the light emitting section 11 does not emit light even upon irradiation with light. In contrast, in Fig. 2, the target capturer is not interacting with an electrically conductive member such as a metal electrode and is released from the electrically conductive member, and the light emitting section 11 emits light upon irradiation with light.

The target capturer can be desorbed from the electrically conductive member, such as a metal electrode, with a controlled timing and can thereby emit light with an arbitrarily controlled timing. The presence or absence, for example, of the target can be easily detected by detecting typically a change in the luminous time of the target capturer, utilizing the phenomenon that the diffusion velocity of the target capturer deceases upon capture of the target.

Thus, the target capturer can be suitably used in the areas in which a variety of targets is detected, analyzed or diagnosed and can be particularly preferably used in the target detecting device mentioned below. The target capturer can be applied to a so-called protein chip. For example, hepatic cells switchover the intracellular glycogen metabolism depending on the condition of receiving insulin in diabetes mellitus. In this case, the protein chip can detect decrease or increase of part of interaction network of a series of proteins from the insulin receptor to the glucogenase and can grasp the population of proteins including so-called posttranslational modifications such as phosphorylation and glycosylation. As a result, the phenomenon that, for example, the hypofunction or hypoactivity of a specific protein relating to the interaction network causes insufficient glucose metabolism can be grasped, which enables an appropriate diagnosis or treatment corresponding to the cause of the malfunction. In contrast, such a phenomenon expressed as a symptom is roughly understood as, for example, diabetes mellitus according to conventional technologies.

### (Target Detecting Device)

The target detecting device comprises a target capturer, means for releasing the target capturer, light irradiating means and light detecting means and may further comprise any other means appropriately selected according to necessity.

### -Target Capturer-

The target capturer is not specifically limited, as long as at least partially comprising a region interactive with an electrically conductive member, is capable of capturing the target and is capable of emitting light upon irradiation with light when not interacting with the electrically conductive member. The target capturer of the present invention is suitably used herein. Details of the target capturer are as described above.

The target capturer is used in the following manner. Specifically, the target capturer is kept as being interacting with the electrically conductive member such as a metal electrode, and the means for releasing the target capturer ceases the interaction to thereby release the target capturer from the electrically conductive member.

The number of the target capturers is not specifically limited and can be appropriately selected according typically to the number and size of the target, and/ or the emission intensity and emission stability of the light emitting section. The number of the target capturers is preferably increased to some extent from the viewpoint of reducing measurement noise.

### -Means for Releasing the Target Capturer -

The means for releasing the target capturer is not specifically limited and can be appropriately selected according to the purpose, as long as it is capable of releasing the target capturer from the electrically conductive member by ceasing the interaction between the target capturer and the electrically conductive member and includes, for example, releasing means and cleaving means.

The releasing means is means for releasing the target capturer from the electrically conductive member by ceasing the interaction between the target capturer and the electrically conductive member. When the interaction between the target capturer and the electrically conductive member is an electrical coupling, suitable examples thereof are a system comprising a pair of electrodes and a power source capable of applying a voltage between the pair of electrodes, in which the pair of electrodes comprise the electrically conductive member as one electrode, and a reference electrode in the electrically conductive substrate; and a system comprising a combination of electrodes and a power source capable of applying, for example, a voltage to the combination of electrodes, in which the combination of electrodes comprising the pair of electrodes and a standard electrode for controlling the electrical potential between the pair of electrodes.

When the said releasing means is used as the means for releasing the target capturer, an electric field is applied to the electrically conductive member, and a reverse electric field to the applied electric field is applied to thereby release the target capturer from the electrically the electrically conductive member by ceasing the interaction therebetween by the action of electrical repulsive force. More specifically, when the interacting section of the target capturer is the polynucleotide serving as a negatively charged anionic polymer, the target capturer can be held by the electrically conductive member by the action of electrical adhesion (interaction) therebetween, as a result of application of a positive electric field to the electrically conductive member such as a metal electrode. In contrast, by applying a negative electric field to the electrically conductive member, the target capturer is released from the electrically conductive member by the action of electrical repulsive force between the electrically conductive member and the target capturer. Thus, simply by appropriately changing the electric field to be applied to the electrically conductive member, the target capturer can be freely and arbitrarily released from the electrically conductive member.

The cleaving means is means for releasing the target capturer from the electrically conductive member by cleaving therebetween. Examples thereof include means for applying stimulus to the target capturer to cleave part of the target capturer to thereby release the interacted target capturer from the electrically conductive member.

The stimulus is not specifically limited and can be appropriately selected according to the purpose. Suitable examples thereof are light or radiation, electricity, agents and enzymes. Each of these can be used alone or in combination.

To use the cleaving means, the target capturer must have a region corresponding to the stimulus applied by the cleaving means and capable of cleaving by the action of the stimulus.

When the interacting section of the target capturer is the polynucleotide, the stimulus is preferably the enzyme such as a restriction endonuclease, and the polynucleotide is cleaved at a specific site (for example, restriction endonuclease site) by the action of the applied enzyme.

### -Light Irradiating Means -

The light irradiating means is not specifically limited, can be appropriately selected according to the purpose, as long as it is capable of applying light radiation to the electrically conductive member, and includes, for example, a ultraviolet lamp and a semiconductor laser system.

The irradiation direction of the light irradiating means is not specifically limited and can be appropriately selected according to the purpose. For example, the light irradiating means preferably applies the light in a direction identical to the surface direction of the electrically conductive member holding the target capturer and in such a direction that reflected light from the electrically conductive member travels in a direction toward the light detecting means.

The wavelength of light applied by the light irradiating means is not specifically limited and can be appropriately selected according the wavelength of absorption peak of the light emitting section, as long as the light emitting section of the target capturer can absorb the light and emit light after absorption.

### -Light Detecting Means -

The light detecting means is not specifically limited and can be appropriately selected according to the purpose, as long as it is capable of detecting light emitted by the target capturer upon irradiation with light applied by the light irradiating means. Each of such light detecting means can be used alone or in combination. Of these light detecting means, a preferred means is one that is capable of receiving light reflected by the electrically conductive member and detecting light emitted from the target capturer, which target capturer is released from the electrically conductive member and diffuses and migrates from inside a detection region toward outside thereof. In this configuration, a light-receivable region is constant, and the light detecting means is capable of detecting the light emitted by the target capturer diffusing and migrating in the light-receivable region. Thus, the light detecting means can detect whether or not the target is captured by the target capturer by determining the diffusion velocity of the target capturer in advance.

Suitable specific examples of the light detecting means are photo-detectors, CCD cameras, photomultipliers and photodiodes.

For efficiently and easily detecting the presence of the target, the light detecting means in the present invention is preferably so configured as to determine the number of photons per unit time emitted by the target capturer by calculation; to detect the intensity of fluorescence emitted by the target capturer; or to determine the time between emission and quenching (becoming not detectable) of the light emitted by the target capturer. More preferably, the light detecting means is so configured as to determine the number of photons per unit time emitted by a target capturer capturing the target, which target capturer is released from the electrically conductive member and diffuses and migrates from inside a detection region toward outside thereof, to determine the number of photons per unit time emitted by a target capturer capturing no target, which target capturer is released from the electrically conductive member and diffuses and migrates from inside a detection region toward outside thereof, and to compare between the numbers of photons to thereby detect whether or not the target capturer captures the target.

This configuration is advantageous to detect or diagnosis of the presence of the target or to determine the amount thereof by previously determining the numbers of photons in the case where the target capturer captures the target and in the case where it does not capture the target and plotting a standard curve.

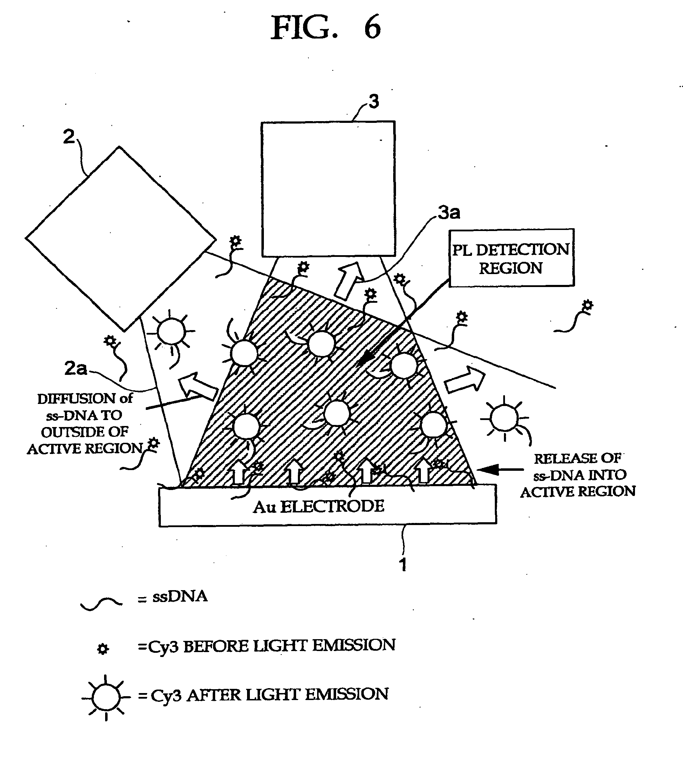

The operation of the target detecting device will be illustrated with reference to the drawings. Fig. 6 is a schematic explanatory diagram showing the target detecting device. This target detecting device at least comprises a target capturer identical to that shown in Figs. 1 and 2, an ultraviolet lamp 2 serving as the light irradiating means, and a photoreceiver 3 serving as the light detecting means. The target capturer is held on a metal electrode 1 serving as the electrically conductive member. The device also includes a reference electrode (not shown) above the metal electrode 1. The metal electrode 1 and the reference electrode are connected to a power source (not shown) capable of applying an electric field and are immersed in a liquid containing a sample mixture. The metal electrode 1, the reference electrode, and the power source for applying an electric field thereto constitute the means for releasing the target capturer.

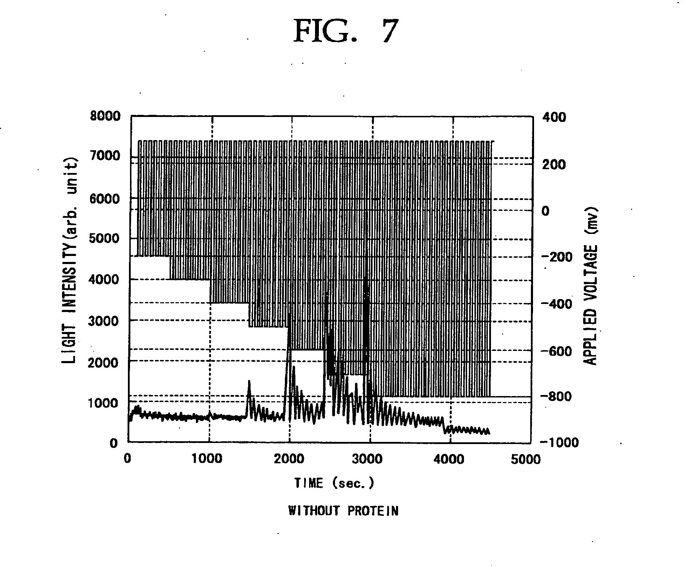

The ultraviolet lamp 2 is so arranged that the light reflected by the metal electrode 1 travels in a direction toward the photoreceiver 3 and serves to apply ultraviolet ray 2a to the surface of the metal electrode 1 (Figs. 6 and 7). The photoreceiver 3 receives the reflected light and receives light emitted by the light emitting section (corresponding to the light emitting section 11 in Figs. 1 and 2) of the target capturer (Figs. 6 and 7).

By actuating the power source as the means for releasing the target capturer to apply a negative or positive electric field to the metal electrode 1, the target capturer is bound to the metal electrode 1 by the action of an electric interaction between the interacting section 10 of the target capturer and the metal electrode 1 (Fig. 3). Under this condition, the target capturer is electrically bound to the metal electrode 1 and does not emit light even upon irradiation with light applied by the light irradiating means. Thus, the photoreceiver 3 does not detect light emitted by the target capturer (Fig. 6).

On the other hand, by actuating the power source to apply an electric field reverse to the electric field already applied to the metal electrode 1, the target capturer is released from the metal electrode by the action of Coulomb repulsive force between the metal electrode 1 and the interacting section 10 of the target capturer (Fig. 4). Under this condition, the target capturer receives the ultraviolet ray 2a applied by the ultraviolet lamp 2, absorbs the light energy of the ultraviolet ray 2a, and the light emitting section 11 thereof emits light (Fig. 7). Then, the photoreceiver 3 detects the reflected light reflected by the metal electrode 1 and also detects the light emitted by the light emitting section 11 of the target capturer.

When the target capturing section of the target capturer is capturing the target, the photoreceiver 3 detects an increased quantity of emitted light (number of photons per unit time) emitted by the target capturer, as compared with the case where the target capturer is not capturing the target, because the target capturer has a decreased molecular diffusion velocity upon capturing of the target. The photoreceiver 3 detects the presence or absence of the target based on the presence or absence of a change in the quantity of emitted light.

As is described above, the target detecting device of the present invention can efficiently detect a variety of targets such as proteins without labeling typically with fluorescence and can thereby be suitably applied to diagnoses of, for example, a variety of diseases such as diabetes mellitus, hypertension, hyperlipidemia, and other multifactorial diseases.

### (Device and Method for molecular adsorption or desorption)

The device for molecular adsorption or desorption comprises two or more adsorption/ desorption electrodes that are controlled independently, and are capable of undergoing at least one of molecular adsorption and molecular desorption and may further comprise any other means such as a counter electrode and a reference electrode appropriately selected according to necessity.

The method for molecular adsorption or desorption comprises the step of applying electric potentials to two or more adsorption/desorption electrodes, the electric potentials arbitrarily varying with different timings, and the step of carrying out one of molecular adsorption and molecular desorption with different timings by the two or more adsorption/ desorption electrodes and may further comprise any other treatment or process appropriately selected according to necessity. The method for molecular adsorption or desorption can be advantageously carried out by using the device for molecular adsorption or desorption.

The device for molecular adsorption or desorption will be illustrated below with the explanation of the method for molecular adsorption or desorption of the present invention.

### -Adsorption/Desorption Electrodes -

The adsorption/ desorption electrodes are not specifically limited in their material, shape, structure, size, surface appearance, can be appropriately selected according to the purpose from among known ones.

Two or more adsorption/ desorption electrodes capable of being independently controlled must be used in the present invention. The two or more adsorption/ desorption electrodes may be the same as or different from each other.

The material is not specifically limited, as long as it has electrically conductive and includes, for example, metals, alloys, electrically conductive resins and carbon compounds.

Examples of the metals are gold, platinum, silver, copper and zinc.

Examples of the alloys are alloys each containing two or more different types of the metals as exemplified above.

Examples of the electrically conductive resins are polyacetylenes, polythiophenes, polypyrroles, poly-p-phenylenes, polyphenylenevinylenes and polyanilines.

Examples of the carbon compounds are electrically conductive carbon and electrically conductive diamond.

Each of these materials can be used alone or in combination. The number of types of metals when used as the material is preferably small. If a metal or metals baser than the metal(s) as the material for the adsorption/ desorption electrodes are brought into electrical contact with the adsorption/ desorption electrodes directly or indirectly typically with the interposition of an electrically conductive liquid, the adsorption/desorption electrodes may be corroded or the adsorption/ desorption electrodes may invite selective adsorption of the molecule.

The shape is not specifically limited, can be selected according to the purpose and includes, for example, a plane, a circular or an elliptic shape. Each of these shapes can be employed alone or in combination.

The structure is not specifically-limited, can be selected according to the purpose and may be a single-layer structure or a multilayer structure.

The size is not specifically limited and can be appropriately selected according to the purpose. For example, the width or diameter is about 500 µm or less and preferably 100 to 300 µm to prepare the device for molecular adsorption or desorption as a chip, and is preferably less than 100 µm to prepare the device for molecular adsorption or desorption as a fine chip. The two or more the adsorption/ desorption electrodes may each have a size identical to or different from each other.

The surface appearance is not specifically limited, can be selected according to the purpose and includes, for example, a glossy surface and a rough surface. The surface is preferably a glossy surface which has been burnished (polished).

The surface of the adsorption/ desorption electrodes for use in the present invention may be coated with a dielectric film so as to expose part of the adsorption/desorption electrodes. Thus, the sizes of the adsorption/ desorption electrodes can be appropriately controlled at a desired level. This configuration is advantageous to enable a downsized, a chip-form and/or integrated device. This is because the sizes, i.e., exposed surfaces of the adsorption/desorption electrodes can be easily and conveniently controlled to a desired level only by preparing the adsorption/ desorption electrodes to suitable sizes for molding, applying the dielectric film to the adsorption/ desorption electrodes and patterning the film, without micromachining of the adsorption/ desorption electrodes.

Upon coating (patterning) of the adsorption/ desorption electrodes to a desired shape and/or a size using the dielectric film, the size of exposed surface of the adsorption/ desorption electrodes is not specifically limited, can be appropriately set according to the purpose. For example, the width or diameter of the exposed surface is about 500 µm or less and preferably 100 to 300 µm to down-size the device for molecular adsorption or desorption, and is preferably less than 100 µm to form the device for molecular adsorption or desorption into a fine chip. The sizes of exposed surfaces of the two or more the adsorption/ desorption electrodes may be the same as or different from each other. The shape of the exposed surfaces of the adsorption/ desorption electrodes is not specifically limited, can be appropriately selected according to the purpose and includes, for example, a substantially rectangular shape, a substantially circular shape and a substantially elliptic shape.

The dielectric film is not specifically limited typically in its material, shape, structure, thickness and size and can be appropriately selected according to the purpose from among know ones.

Examples of the material are silicon nitride and a resist material. Each of these materials can be used alone or in combination. Among them, a resist material is preferred for easily processing the work into a fine and complicated shape and is advantageous for the down-sizing, forming into a chip and/or integration of the device.

The resist material is not specifically limited and can be appropriately selected according to the purpose from among known materials. From the viewpoint of fine patterning, the resist material is preferably at least one selected from, for example, g-line resists, i-line resists, KrF resists, ArF resists, F2 resists and electron beam resists. Each of these resist materials can be used alone or in combination.

The shape is not specifically limited, can be selected according to the purpose and includes, for example, various patterns. The structure is not specifically limited, can be selected according to the purpose and may be a single-layer structure or a multilayer structure. The thickness and the size are not specifically limited and can be appropriately selected according to the purpose.

The dielectric film can be prepared according to a known method including vapor deposition such as CVD or PVD; or coating.

When the dielectric film uses the resist material as the material, the dielectric film having a desired patterned shape can be prepared by applying and drying the resist material to form a resist film and pattering the resist film into a desired shape typically by exposure and development.

The interval between exposed areas of adjacent two or more adsorption/ desorption electrodes is not specifically limited and can be appropriately selected according to the purpose. The distance is preferably, for example, 100 nm or more while varying depending on the concentration of after-mentioned electrolyte, from the viewpoint of not adversely affecting molecules adsorbed by the adjacent adsorption/ desorption electrodes.

The adsorption/ desorption electrodes may be suitably prepared or be commercially available products. A method for preparing the adsorption/ desorption electrodes is not specifically limited, can be selected according to the purpose and includes, for example, vapor deposition such as CVD or PVD; lead-free plating; and sputtering. Each of these methods can be carried out alone or in combination.

The number of the adsorption/ desorption electrodes is not specifically limited, as long as it is two or more and can be appropriately set according typically to the type of molecule to be adsorbed or desorbed. For a chip for diagnosis and/or analysis, for example, the number is preferably three or more, and more preferably four or more.

The two or more adsorption/desorption electrodes can be used as being integrated with a substrate or being independent from the substrate.

When the adsorption/desorption electrodes are integrated with the substrate, they can be integrated with the same substrate or with different substrates.

The two or more adsorption/ desorption electrodes integrated with the same substrate are advantageous for forming the device for molecular adsorption or desorption into a chip. The two or more adsorption/desorption electrodes integrated with different substrates are advantageous for enhancing, for example, the permanence, durability and/or mechanical strength of the adsorption/desorption electrodes.

When the two or more adsorption/ desorption electrodes are integrated with the same and one substrate, the one substrate must have two or more of the adsorption/ desorption electrodes. When the two or more adsorption/ desorption electrodes are integrated with the same two substrates, each of the substrates comprises preferably two or more of the adsorption/ desorption electrodes, more preferably three or more, and further preferably four or more of the adsorption/ desorption electrodes from the viewpoint of forming the device for molecular adsorption or desorption into a chip

The two or more adsorption/desorption electrodes are preferably so designed as to endure repetitive use, when they are integrated with different substrates, namely, when they are used independently from each other.

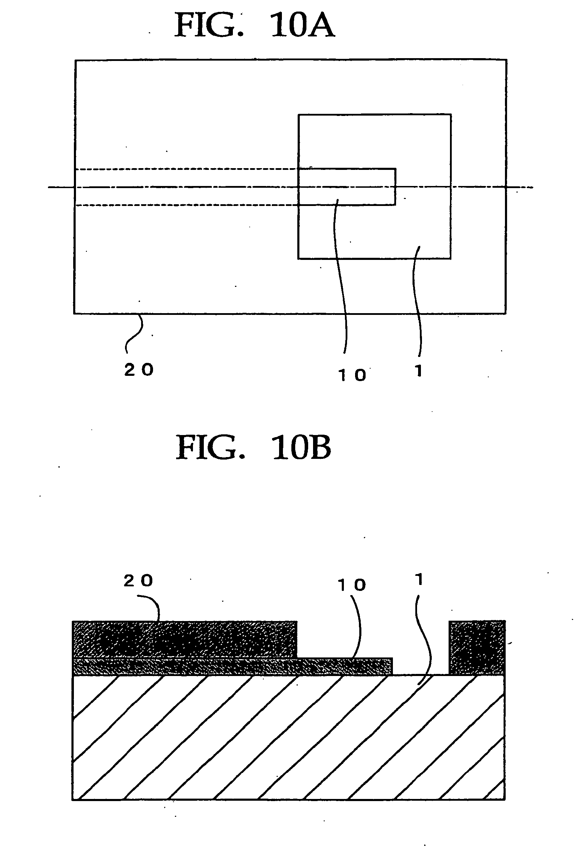

This aspect is shown in Figs 10A and 10B. With reference to Figs. 10A and 10B, an adsorption/ desorption electrode 10 is integrated and fixed with a substrate 1. Figs. 10A and 10B are a plan view and a side view, respectively. The substrate 1 herein is a silicon oxide substrate. The adsorption/ desorption electrode 10 is a rectangular thin-plate gold electrode. An adhesion layer (not shown), mentioned later, is arranged between the adsorption/ desorption electrode 10 and the substrate 1 for bringing them into intimate contact with each other. The adhesion layer comprises chromium. The surface of the adsorption/ desorption electrode 10 is coated with a dielectric film 20 so that one end of the adsorption/ desorption electrode 10 and part of the surface of the substrate 1 are exposed therefrom. The exposed area of the adsorption/ desorption electrode 10 and the substrate 1 constitutes a site in which adsorption or desorption of the molecule is carried out.

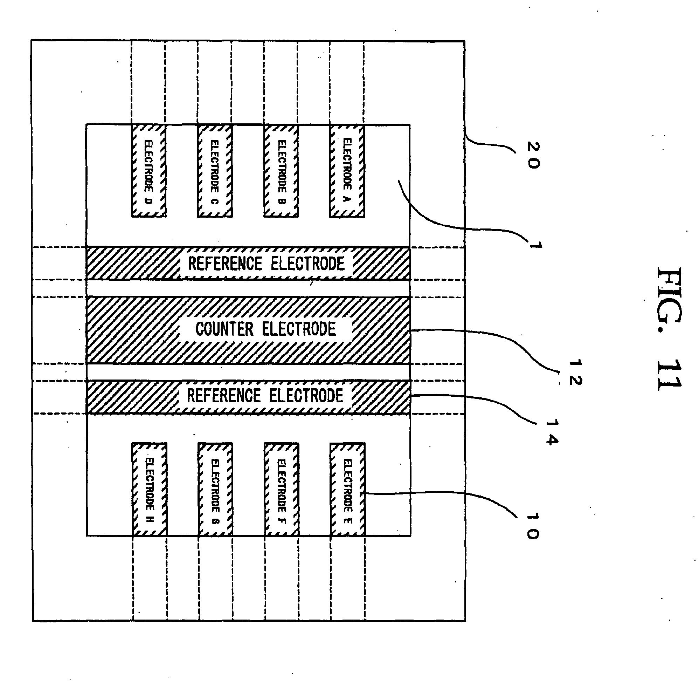

In Figs.10A and 10B, the one substrate 1 has one adsorption/ desorption electrodes 10, but one substrate 1 may have two or more adsorption/ desorption electrodes 10 (Fig. 11). The exposed area typically of the adsorption/ desorption electrodes 10 is not specifically limited, can be selected according to the purpose may be at an end part or at a center part of the adsorption/ desorption, electrode 10.

The substrate is not specifically limited typically in its shape, structure, thickness and size can be appropriately selected according to the purpose from among known substrates.

Suitable examples of the substrate are insulating substrates. The insulating substrates include, for example, a quartz glass substrate, silicon substrate, silicon oxide substrate, silicon nitride substrate, and sapphire substrate. Each of these can be used alone or in combination.

When the two or more adsorption/ desorption electrodes are integrated with the same substrate, the device for molecular adsorption or desorption of the present invention may have two or more pieces of the substrate. This configuration is advantageous for yielding the adsorption/ desorption device as a high-performance, high-efficiency integrated adsorption/ desorption device and for improving detection efficiency and/or detection sensitivity typically in diagnosis and/or analysis. The numbers of the adsorption/desorption electrodes in the individual substrates may be the same as or different from each other.

The latter aspect is shown in Fig.11. With reference to Fig.11, a total of eight adsorption/desorption electrodes 10 (Electrode A, Electrode B, Electrode C, Electrode D, Electrode E, Electrode F, Electrode G and Electrode H) are integrated and fixed to a substrate 1. The integrated adsorption/ desorption device has nine substrates. Each of the substrates 1 has one rectangular counter electrode 12 and two rectangular reference electrodes 14, each integrated and fixed thereto. Thus, the counter electrode 12 is arranged linearly at the center part of the substrate 1 along a transverse direction, and the reference electrodes 14 are arranged linearly along the transverse direction so as to sandwich the counter electrode 12. Each four adsorption/ desorption electrodes 10 (Electrode A, Electrode B, Electrode C and Electrode D, and, Electrode E, Electrode F, Electrode G and Electrode H) are arranged at both ends of the substrate 1, respectively, so as to sandwich and face the reference electrodes 14.

The substrate 1 herein is a quartz glass substrate. The adsorption/ desorption electrodes 10 are thin-plate gold electrodes as in Figs.10A and 10B. The counter electrode 12 and the reference electrodes 14 are Ag/AgCl alloy electrodes. An adhesion layer (not shown) mentioned later is arranged between the substrate 1 and the adsorption/ desorption electrodes 10, the counter electrode 12 or the reference electrodes 14 for bringing them into intimate contact with each other. The adhesion layer comprises chromium. A dielectric film 20 is applied to the surface of the adsorption/desorption electrodes 10, the counter electrode 12 and the reference electrodes 14 so as to expose one end of each of the adsorption/ desorption electrodes 10, a portion of the counter electrode 12 other than both ends, a portion of the reference electrodes 14 other than both ends, and part of the substrate 1 therefrom. The exposed area of the adsorption/desorption electrodes 10, the counter electrode 12, the reference electrodes 14 and the substrate 1 constitutes a site where the adsorption or desorption of the molecule is carried out The adsorption/desorption electrodes 10, the counter electrode 12 and the reference electrodes 14 are connected to power sources (not shown) in a conductive manner, and Electrode A, Electrode B, Electrode C, Electrode D, Electrode E, Electrode F, Electrode G and Electrode H in the adsorption-release electrodes 10 can be driven independently.

The integrated adsorption/ desorption device will be illustrated below.

The integrated adsorption/ desorption device is not specifically limited and, can be appropriately designed according to the purpose and is preferably so configured that each of substrates has a total of n adsorption/ desorption electrodes comprising first, second,....... (n-1)th and (n)th adsorption/ desorption electrodes, and wherein the first adsorption/ desorption electrodes in the respective substrates are electrically connected to an identical power source, the second adsorption/ desorption electrodes are electrically connected to an identical power source,....... the (n-1)th adsorption/desorption electrodes are electrically connected to an identical power source, and the (n)th adsorption/desorption electrodes are electrically connected to an identical power source, respectively. This configuration can treat a multiplicity of diagnosis and/ or analysis samples at once with high efficiency.

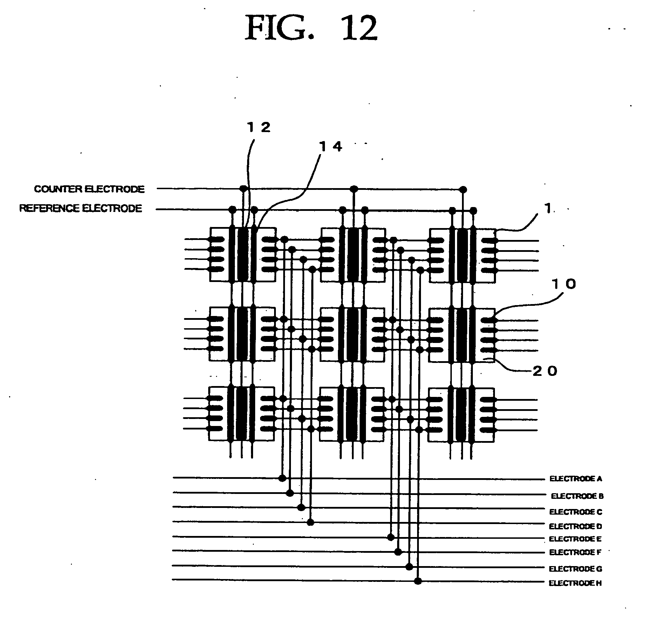

This aspect is shown in Fig. 12. The integrated adsorption/desorption device includes nine rectangular substrates. Each of the substrates comprises eight adsorption/desorption electrodes (Electrode A, Electrode B, Electrode C, Electrode D, Electrode E, Electrode F, Electrode G, and Electrode H), one counter electrode and the reference electrodes, each integrally fixed thereto. Each of the substrates has the counter electrode and the reference electrodes so as to sandwich the counter electrode. More specifically, the counter electrode is arranged linearly at the center part of the substrate along a transverse direction in Fig. 12, and the reference electrodes are arranged linearly along the transverse direction. Each four adsorption/ desorption electrodes) are arranged at one end of, i.e. a total of eight adsorption-release electrodes at both ends of, the substrate, so as to sandwich and face the reference electrodes. The individual substrates have fundamentally the same structure as that shown in Fig.11.

The nine substrates are arranged each three in three rows at substantially identical intervals, and the individual substrates in each row have the same configuration as one another. In Fig. 12, the respective substrates arranged in the left and right rows have the same configuration as one another and have Electrode E, Electrode F, Electrode G and Electrode H in this order from the left top end, and Electrode A, Electrode B, Electrode C and Electrode D in this order from right top end. The respective substrates in the central row have the same configuration as one another, respective substrates and have Electrode A, Electrode B, Electrode C and Electrode D arranged in this order from the left top end, and Electrode E, Electrode F, Electrode G and Electrode H in this order from the right top end.

Electrodes A, B, C and D in the respective substrates in the left row face Electrodes A, B, C and D in the respective substrates in the central row arrange, respectively. Likewise, Electrodes E, F, G and H in the respective substrates in the central row face Electrodes E, F, G and H in the respective substrates in the right row, respectively.

The counter electrodes in the respective substrates are connected through an identical lead wire and are capable of being driven by an identical power source. Likewise, each of the reference electrodes, each of Electrodes A, each of Electrodes B , each of Electrodes C, each of Electrodes D, each of Electrodes E, each of Electrodes F, each of Electrodes G, and each of Electrodes H are connected through an identical lead wire and are capable of being driven by an identical power source, respectively.

In the integrated adsorption/ desorption device, by controlling voltages and thereby changing electric potentials between Electrodes A and the counter electrodes in the nine substrates by the action of operating the power source for controlling Electrodes A, the molecule can be adsorbed or desorbed only in the Electrode A portion. In contrast, the other Electrodes B, C, D, E, F, G and H portions, the molecule is not adsorbed or desorbed since there is no change in electric potential. In this configuration, nine different samples can be simultaneously analyzed and/ or diagnosed, and eight different molecules corresponding to Electrodes A, B, C, D, E, F, G and H can be adsorbed or desorbed. The device is therefore of high efficiency and of high performance.

When the adsorption/ desorption electrodes are integrated and fixed to the substrate in the present invention, an adhesion layer is preferably arranged between the substrate and the adsorption/ desorption electrodes for enhancing adhesion between the two.

The adhesion layer is not specifically limited typically in its material, shape, structure, thickness and size and can be appropriately selected according to the purpose.

Examples of the material are chromium, and platinum/ titanium.

The structure is not specifically limited and, can be selected according to the purpose and may be a single-layer structure or a multilayer structure.

The thickness is not specifically limited and can be appropriately selected according to the purpose.

The size is not specifically limited and can be appropriately set according typically to the size of the adsorption/desorption electrodes.

Each of the adsorption/ desorption electrode is connected typically to a power source so as to be capable of changing the electric potential thereof. It is preferably so configured that the electric potential can be varied from a positive electric potential to a negative electric potential. This configuration advantageously enables the molecule to be reversibly adsorbed and desorbed by the adsorption/ desorption electrode. More specifically, by applying a negative electric potential to the adsorption/ desorption electrode which has been applied with a positive electric potential, the molecule adsorbed by the adsorption/ desorption electrode can be desorbed, or the molecule liberated can be adsorbed by the adsorption/ desorption electrode.

At least two of the two or more adsorption/ desorption electrodes are preferably connected to, for example, different power sources for independently controlling two or more of the adsorption/ desorption electrodes. More preferably, the respective adsorption/ desorption electrodes are connected to, for example, different power sources, respectively, so as to independently control the two or more adsorption/ desorption electrodes independently of each other.

The device for molecular adsorption or desorption of the present invention preferably further comprises a counter electrode for forming an electric circuit with the two or more adsorption/ desorption electrodes. The counter electrode is used to constitute an electric circuit with the adsorption/desorption electrodes to thereby balance an electric current budget

The counter electrode is not specifically limited and, can be selected according to the purpose from known ones and includes, for example, those listed as the adsorption/ desorption electrodes. The number of types of the metals for the counter electrode is preferably few, as in the adsorption/desorption electrodes.

The counter electrode may be integrated and fixed to the substrate with the adsorption/ desorption electrodes, may be integrated and fixed to another substrate than the substrate or may be used as an independent part.

The number of the counter electrode is not specifically limited and can be appropriately set according to the purpose, but is preferably few. When two or more adsorption/ desorption electrodes are arranged per one substrate, each substrate preferably has one counter electrode. In this case, the two or more the adsorption/desorption electrodes are preferably arranged so as to face the counter electrode and are more preferably arranged at both edges of the substrate so as to face and sandwich the counter electrode arranged at a center part of the substrate.

The device for molecular adsorption or desorption preferably further comprises one or more reference electrodes in addition to the counter electrode. This configuration constitutes a so-called three-electrode control and enables easier control of the electric potential between the adsorption/desorption electrodes and the counter electrode than a two-electrode control without using the reference electrode. The reference electrode is advantageously usable for determining or observing a standard electric potential.

The reference electrode is not specifically limited, can be selected according to the purpose from known ones and includes, for example, those listed as the adsorption/ desorption electrodes. The number of types of material metals for the reference electrode is preferably few, as in the adsorption/ desorption electrodes.

The reference electrode may be integrated and fixed to the substrate together with at least one of the adsorption/desorption electrodes and the counter electrode, may be integrated and fixed to another substrate than the substrate or may be used as an independent part.

The number of the reference electrode is not specifically limited, can be appropriately selected according to the purpose but is preferably few. When each of the substrates has one counter electrode, it preferably has two reference electrodes. In this case, the reference electrodes are preferably arranged so as to sandwich the counter electrode, and more preferably, the adsorption/ desorption electrodes are arranged at edges of the substrate so as to sandwich the reference electrodes.

The molecule is not specifically limited and can be appropriately selected according to the purpose, as long as it is interactive with the adsorption/ desorption electrodes.

The interaction is not specifically limited, can be appropriately selected according to the purpose and includes, for example, electrical actions such as electrical coupling; chemical actions such as chemical binding; and physical actions such as adsorption. Among them, electrical actions such as electrical coupling are preferred, from the viewpoint of enabling the adsorption/desorption device to reversibly adsorb and desorb the molecule.

The size of an interactive region is not specifically limited, can be appropriately set according typically to the intensity of the interaction and is preferably large, from the viewpoint of reliably adsorbing or desorbing the molecule by the adsorption/ desorption electrodes.

The interactive region may partially comprise sites having different interactivities, such as associative strength, with the adsorption/desorption electrodes.

The number of the interactive region per one molecule is not specifically limited, can be appropriately selected according to the purpose, is at least one and may be two or more.

The shape of the molecule is not specifically limited, can be selected according to the purpose and includes, for example, a linear shape (filamentous shape), a granular shape, a plate shape, and a combination of two or more of these shapes, of which a liner or filamentous shape is preferred.

The molecule is not specifically limited and can be selected according to the purpose. Suitable examples of the molecule are biomolecules, from the viewpoint of applying typically to treatment and/ or diagnosis of diseases. The molecule is preferably a charged molecule, since such a charged molecule can electrically interact with the adsorption/ desorption electrodes. Each of these molecules can be used alone or in combination.

The charged molecule is not specifically limited, can be appropriately selected according to the purpose, and suitable examples thereof are ionic polymers.

The ionic polymer is preferably selected from cationic polymers and anionic polymers.

Suitable examples of the cationic polymers (positively charged ionic polymers) are guanidine DNA and polyamines.

Suitable examples of the anionic polymers (negatively charged ionic polymers) are polynucleotides and poly(phosphoric acid)s. These substances are preferred, since they have negative charges dispersed at constant intervals in the molecule and serve to easily control the interaction, such as coupling or binding, with the adsorption/desorption electrodes.

Of the polynucleotides, preferred are DNAs, RNAs, and complexes of these with proteins. The DNAs and RNAs may each be single-stranded or double-stranded.

Specific examples of the polynucleotide are cancer-associated genes, genes relating to hereditary diseases, viral genes, bacterial genes and genes showing polymorphism and called as disease risk factors.

Examples of the cancer-associated genes are k-ras gene, N-ras gene, p53 gene, BRCA1 gene, BRCA2 gene, src gene, ros gene and APC gene.

Examples of the genes relating to hereditary diseases are genes relating to inborn errors of metabolism, such as phenylketonuria, alcaptonuria, cystinuria, Huntington chorea, Down syndrome, Duchenne muscular dystrophy and haemophilia.

Examples of the viral genes and the bacterial genes are genes of hepatitis C virus, hepatitis B virus, influenzae viruses, measles virus, HIV virus, mycoplasma, rickettsia, streptococci and Salmonella typhimurium.

Examples of the genes showing polymorphism include genes having different base sequences from individual to individual and being not always directly related to causes of diseases, such as PS1 (presenilin-1) gene, PS2 (presenilin-2) gene, APP (amyloid beta precursor protein) gene, lipoprotein gene, genes relating to HLA (human leukocyte antigen) or blood typing, and genes believed to be involved in the onset of, for example, hypertension or diabetes.

A method for preparing the polynucleotide is not specifically limited, can be appropriately selected from known methods according to the purpose and includes, for example, a method using a DNA synthesizer (DNA automatic synthesis system); a method in which an oligonucleotide sequence previously prepared is combined by the action of a primer and a DNA polymerase; and a method in which an oligonucleotide sequence previously prepared is subjected to annealing with arrayed oligomer blocks, and a DNA ligase or an RNA ligase is allowed to react to thereby bind them.

The length of the polynucleotide is not specifically limited, can be appropriately selected according to the purpose and is preferably at least six bases. A peak voltage required for allowing the adsorption-release electrode to desorb the polynucleotide generally deceases with a decreasing length of the polynucleotide.

The molecule is desorbed from or adsorbed by the adsorption/desorption electrode, by varying the electric potential of the adsorption-release electrode. For example, a negative electric potential is applied to the adsorption-release electrode which has been applied with a positive electrode. Alternatively, a positive electric potential is applied to the adsorption-release electrode which has been applied with a negative electric potential. When the molecule is, for example, a polynucleotide (DNA), the polynucleotide (DNA) is adsorbed by the adsorption/ desorption electrodes by applying a positive electric potential to the adsorption/desorption electrode, and, inversely, is desorbed from the adsorption/desorption electrode by applying a negative electric potential to the adsorption-release electrode.

The number of the molecule to be adsorbed by or desorbed from the adsorption/desorption electrode is not specifically limited and can be appropriately selected according to the purpose. In the present invention, the two or more adsorption/ desorption electrodes are preferably so configured as to adsorb and/or desorb two or more different molecules from each other. This configuration advantageously enables the adsorption/desorption device to carry out, for example, diagnosis efficiently.

The molecule preferably has a target capturing section capable of capturing a target, from the viewpoint of applying the device typically to diagnosis or analysis.

Suitable examples of the target capturing section are antibodies, antigens, enzymes and coenzyme, against or with respect to the target. The target capturing section can be selected according to the type of the target. When the target is, for example, an antigen, an antibody against the antigen can be selected as the target capturing section. When the target is an antibody, an antigen against the antibody can be selected as the target capturing section. When the target is an enzyme such as avidin, a coenzyme to the enzyme, such as biotin, can be selected as the target capturing section. When the target is a coenzyme such as biotin, an enzyme to the coenzyme, such as avidin can be selected as the target capturing section.

The number of the target capturing section per one molecule of the target capturer is not specifically limited, can be appropriately selected according to the purpose is at least one and may be two or more.

The position of the target capturing section in the target capturer is not specifically limited and can be appropriately selected according to the purpose. When the interacting section is linear or filamentous, the target capturing section may be arranged at the end or terminal of the interacting section. When the interacting section is a polynucleotide, the position may be three prime-end and/or five prime-end.

The target is not specifically limited, can be appropriately selected according to the purpose and includes, for example, organic molecules.

Suitable examples of the organic molecules are proteins, plasma proteins, tumor markers, apoproteins, viruses, autoantibodies, congealing fibrinogenolysis factors, hormones, medicaments or drugs in the blood, nucleic acids, HLA antigens, lipoproteins, glycoproteins, polypeptides, lipids, polysaccharides and lipopolysaccharides.

Examples of the protein are enzymes such as avidin.

Examples of the plasma proteins are immunoglobulins (IgG, IgA, IgM, IgD and IgE), components of complement (C3, C4, C5 and C1q), CRP, α₁ -antitrypsin, α₁ -microglobulin, β₂ -microglobulin, haptoglobin, transferrin, ceruloplasmin and ferritin.

Examples of the tumor markers are α-fetoprotein (AFP), carcinoembryonic antigen (CEA), CA 19-9, CA 125, CA 15-3, SCC antigen, prostatic acid phosphatase (PAP), PIVKA-II, γ-seminoprotein, TPA, elastase I, neuron specific enolase (NSE) and immunosuppressive acidic protein (IAP).

Examples of the apoproteins are Apo A-I, Apo A-II, Apo B, Apo C-II, Apo C-III and Apo E.

Examples of the viruses are hepatitis B virus (HBV), hepatitis C virus (HBC), HTLV-I and HIV. Causes of infective diseases other than viruses include, for example, ASO, toxoplasma, mycoplasma and STD.

Examples of the autoantibodies are anti-microsome antibodies, anti-thyroglobulin antibodies, antinuclear antibodies, rheumatoid factors, antimitochondrial antibodies and antimyelin antibodies.

Examples of the congealing fibrinogenolysis factors are fibrinogen, fibrin decomposition products (FDP), plasminogen, α₂ -plasmin inhibitor, antithrombin III, β-thromboglobulin, Factor VIII, protein C and protein S.

Examples of the hormones are pituitary hormones such as LH, FSH, GH, ACTH, TSH and prolactin; thyroid hormones such as T₃, T₄ and thyroglobulin; calcitonin; parathormone (PTH); adrenal cortical hormones such as aldosterone and cortisol; gonadal hormones such as hCG, oestrogen, testosterone and hPL; hormones of the pancreas and gastrointestinal tract such as insulin, C-peptide, glucagon and gastrin; and other hormones such as renin, angiotensin I, angiotensin II, enkephalin and erythropoietin.

Examples of the medicaments or drugs in the blood are antiepileptic agents such as carbamazepine, primidone and valproic acid; agents for cardiovascular diseases, such as digoxin, quinidine, digitoxin and theophylline; and antibiotics such as gentamicin, kanamycin and streptomycin.

The nucleic acids include those listed above.

The molecule preferably has a light emitting section that is capable of emitting light upon irradiation with light when the molecule is not interacting with the adsorption/ desorption electrode, or a light emitting section that is capable of emitting light upon irradiation with light when the molecule is interacting with the adsorption/ desorption electrode. This configuration enables the diagnosis, for example, by visual observation.

The light emitting section is not specifically limited, can be appropriately selected according to the purpose and suitable examples thereof are fluorescent dyes, metals and semiconductive nanospheres.

The fluorescent dyes are typically preferred as the light emitting section. When the adsorption/ desorption electrode is a metal such as a metal electrode, such a fluorescent dye does not emit light even upon irradiation with radiation having such a wavelength that the fluorescent dye can absorb when the fluorescent dye is interacting with the metal (for example, the fluorescent dye is positioned in the proximity to the metal). On the other hand, the fluorescent dye can emit light by the action of light energy upon irradiation with radiation having such a wavelength that the fluorescent dye can absorb, when the dye is not interacting with the metal (for example, the fluorescent dye is positioned away from the metal).

The fluorescent dye is not specifically limited, can be appropriately selected according to the purpose from among known ones, and suitable examples thereof are compounds represented by following Structural Formula 1:

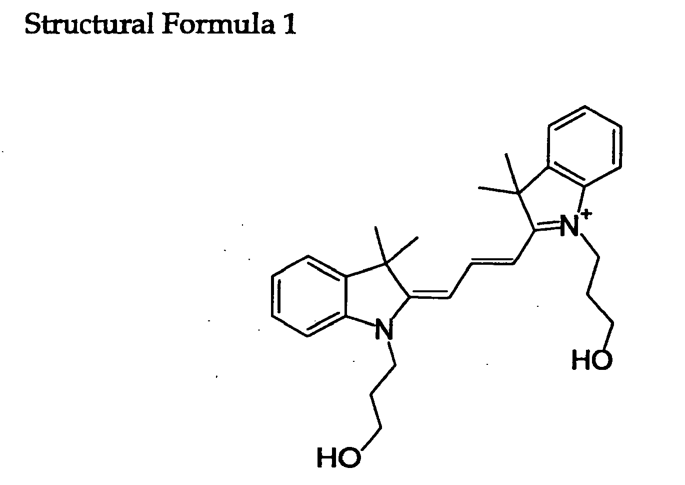

The number of the light emitting section per one molecule of the molecule is not specifically limited, can be appropriately selected according to the purpose, is at least one and may be two or more.

The position of the light emitting section in the molecule is not specifically limited and can be appropriately set according to the purpose. When the interactive region is linear, the light emitting section may be located at an end of the linear interactive region. When the interactive region is a polynucleotide, it may be located at three prime-end and/ or five prime-end.

When the interactive region in the molecule is, for example, the polynucleotide, the molecule can be prepared, for example, in the following manner. Specifically, the polynucleotide strand of an oligo-deoxyribonucleotide modified with a molecule serving as the target capturing section at the five prime-end expands using a primer and DNA polymerase to thereby allow a molecule serving as the light emitting section to bind with the molecular chain.

The adsorption/ desorption electrodes are preferably used, for example, as being immersed in an electrically conductive liquid that does not decompose the molecule. The use of the electrically conductive liquid facilitates the molecular adsorption or desorption.

The electrically conductive liquid is not specifically limited, can be appropriately selected according to the purpose, as long as it is electrically conductive, and includes, for example, water, ion solutions, and solutions each containing an electrolyte. Each of these can be used alone or in combination.

Readsorption or redesorption of the molecule can be prevented by replacing the electrically conductive liquid with a non-conductive liquid after the adsorption or desorption of the molecule.

The non-conductive liquid is not specifically limited, can be appropriately selected according to the purpose and is preferably one that does not deteriorate, for example, the molecule and the adsorption/desorption electrodes. Suitable examples thereof are known organic solvents such as alcohols. Among the organic solvents, alcohols are preferred, of which ethanol is more preferred. By using ethanol as the organic solvent, the liquid can be effectively prevented from putrefaction preclusion and can be conserved over a long term.

The principle of adsorption or desorption of the molecule using the device for molecular adsorption or desorption of the present invention, namely, the principle of adsorption or desorption of the molecule in the method for molecular adsorption or desorption of the present invention will be illustrated below.

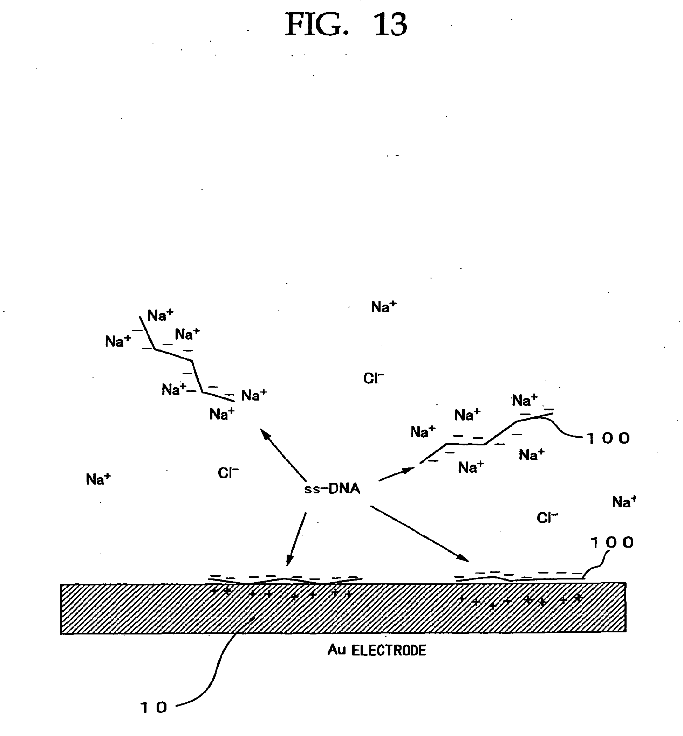

With reference to Fig. 13, an adsorption/desorption electrode 10 and the counter electrode is immersed in a sample containing a DNA molecule 100 as the molecule. The adsorption/ desorption electrode 10 comprises chemically inert Au and is arranged on an insulating substrate (not shown). The sample is an aqueous sodium chloride solution and is the electrically conductive liquid. By applying a positive voltage to the adsorption/ desorption electrode 10 (gold electrode in this embodiment) by a power source (not shown) to thereby apply a positive electric potential thereto, the DNA molecule 100 as the molecule, being negatively charged, is electrically attracted and adsorbed by the adsorption/desorption electrode 10. The adsorption/ desorption electrode 10 constitutes an electric circuit with the counter electrode (not shown) arranged in the aqueous sodium chloride solution. The DNA molecule 100 is rigidly adsorbed by the adsorption/ desorption electrode 10 by the action of electrical attraction and is not desorbed therefrom unless the electric potential of the adsorption/ desorption electrode is changed.

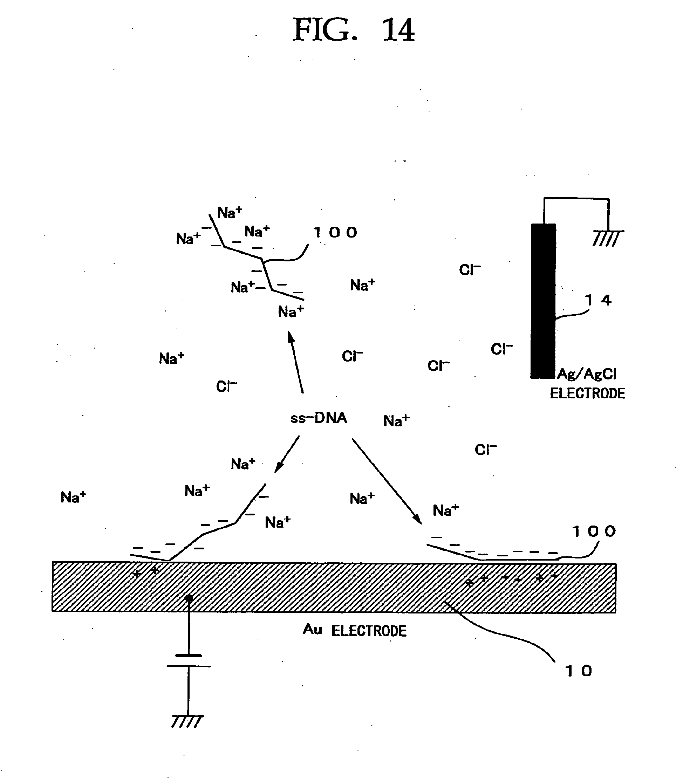

A reference electrode 14 may further be arranged in the electrically conductive liquid to control the electric potential of the adsorption/ desorption electrode 10 to a desired extent (Fig. 14).

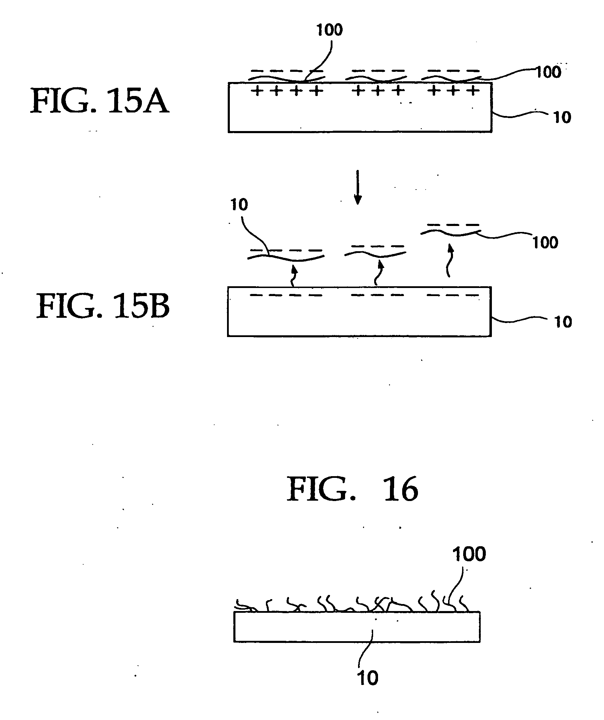

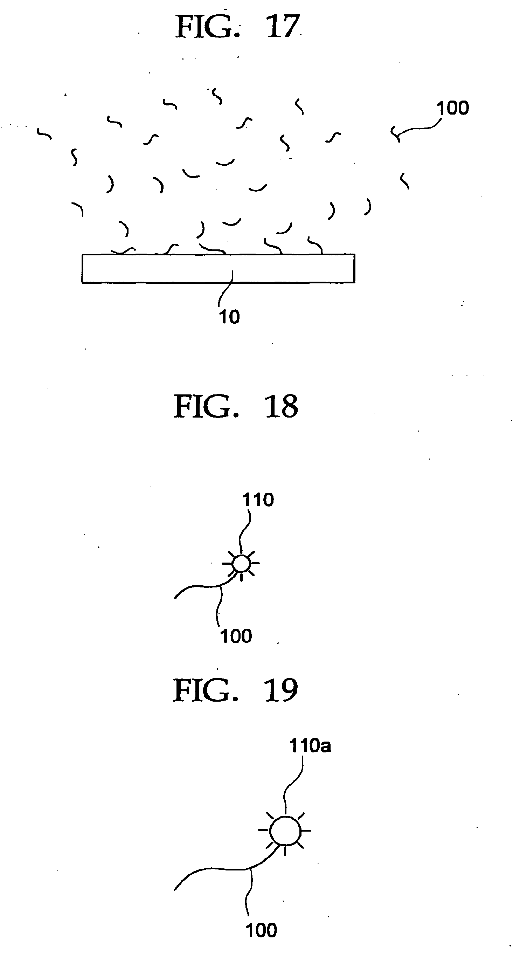

The DNA molecule 100 electrically adsorbed by the adsorption/ desorption electrode 10 (Fig. 15A or Fig. 16) is then desorbed from the adsorption/ desorption electrode 10 by the action of electrical repulsive force (Fig. 15B or Fig. 17) by applying a negative voltage to the adsorption/ desorption electrode 10 by the power source to thereby apply a negative electric potential thereto. There is a threshold electric potential in the electric interaction between the adsorption/ desorption electrode and the molecule depending on the types of the adsorption/ desorption electrode and the molecule. Such an electric potential must be applied to the adsorption/ desorption electrode as to exceed the threshold, in order to allow the adsorption/ desorption electrode to desorb the adsorbed molecule.

The adsorption/ desorption electrode 10 is allowed to repeatedly adsorb and desorb the DNA molecule 100 by repeatedly applying a positive voltage and a negative voltage to adsorption/ desorption electrode 10 from the power source. More specifically, DNA molecule 100 can be reversibly adsorbed and desorbed. Further, the DNA molecule 100 serving as the molecule can be selectively adsorbed by or desorbed from two or more adsorption/ desorption electrodes 10 with different timings, by arbitrarily applying the electric potentials thereto, which electric potentials varying with different timings. In this configuration, no electric potential or a weak electric potential lower than the threshold can be applied to other adsorption/ desorption electrodes 10 than the adsorption/ desorption electrode 10 to which the electric potential exceeding the threshold is applied so as to adsorb or.desorb the DNA molecule 100 as the molecule.

When the molecule includes two or more different molecules, the two or more different molecules can be selectively desorbed with different timings, by allowing different adsorption/ desorption electrodes 10 to adsorb the two or more different molecules respectively. In contrast, the two or more different molecules can be selectively adsorbed by different adsorption/ desorption electrodes with different timings, by allowing the sample to contain the two or more different molecules. A device comprising two or more adsorption/desorption electrodes 10 adsorbing the different molecules can be used as a chip for supplying molecules.

The molecule adsorbed by the adsorption/desorption electrode 10 can be stably stored as intact by allowing the adsorption/ desorption electrode 10 to adsorb DNA molecule 100 and then replacing the electrically conductive liquid with the non-conductive liquid such as an organic solvent. This article can be satisfactorily handled and operated when downsized, formed into a chip and/or integrated. The non-conductive liquid is preferably one that does not deteriorate the DNA molecule 100, for stable preservation over a long time. In this configuration, the DNA molecule 100 serving as the molecule is not desorbed from the adsorption/ desorption electrode 10 even when the power source which has applied the voltage to the adsorption-release electrode 10 is turned off, unless an inverse electric potential is applied to the adsorption-release electrode 10. After replacing the non-conductive liquid with the electrically conductive liquid, the DNA molecule 100 can be desorbed at the time when an inverse electric potential is applied to the adsorption/ desorption electrode 10 to thereby change the electric potential of the adsorption/ desorption electrode.

A lubricant can be applied to the surfaces of the other members than the adsorption/ desorption electrodes, such as the dielectric film and the substrate for preventing the molecule, such as a DNA molecule from being adsorbed by the members.

The lubricant is not specifically limited, can be selected according to the purpose, and suitable examples thereof are fluorine-containing lubricants such as perfluoro-polyether compounds. Each of these can be used alone or in combination.

By applying a voltage to the adsorption/ desorption electrodes, the lubricant is removed from the surfaces of the adsorption/desorption electrodes and is applied to the surfaces of the other members.

Next, molecular adsorption or desorption in the integrated device for molecular adsorption or desorption shown in Fig. 12.

For example, the respective substrates 1 are initially immersed in the electrically conductive liquid. Of adsorption/ desorption electrodes 10 integrated and fixed to the respective substrates 1, a negative voltage exceeding the threshold is applied to the other adsorption/desorption electrodes 10 (Electrodes B, C, D, E, F, G and H) than Electrode A, and a positive voltage is applied only to Electrode A. At this time, a DNA molecule serving as the molecule (hereinafter referred to as "molecule A") is added to the electrically conductive liquid using a dropping pipette from outside and is then adsorbed by Electrode A alone. Thereafter, the electrically conductive liquid is removed to thereby remove the DNA molecule (molecule A) contained in the electrically conductive liquid. The DNA molecule (molecule A) may be adhered to the surfaces of the counter electrode 12 and the reference electrodes 14 fixed to the substrate 1. Such DNA molecule (molecule A) adsorbed by the counter electrode 12 and the reference electrodes 14 can be removed by inserting another electrode into the electrically conductive liquid, and applying a negative voltage to the counter electrode 12, the reference electrodes 14 and the other adsorption/ desorption electrodes than Electrode A, at the time when the electrically conductive liquid is removed. When the counter electrode 12 and the reference electrodes 14 are not integrated and fixed to the substrate 1, the counter electrode 12 and the reference electrodes 14 may be replaced with new ones.

Next, the substrate 1 is immersed in a fresh electrically conductive liquid (electrolyte solution). No voltage or a weak negative voltage equal to or lower than the threshold is applied to Electrode A, a negative voltage equal to or more than the threshold is applied to the other adsorption/desorption electrodes than Electrode A, and a positive voltage is applied only to Electrode B. Under this condition, a DNA molecule (molecule B) is added to the electrically conductive liquid in the same manner as above, and Electrode B can thereby adsorb the DNA molecule (molecule B).

DNA molecules (molecules C, D, E, F, G and H) can be adsorbed by the other adsorption/desorption electrodes, i.e., Electrodes C, D, E, F, G and H, respectively, according to the above-mentioned procedure.

The device for molecular adsorption or desorption enables the adsorption/ desorption electrodes to adsorb the molecules in the above-mentioned manner, does not require the use of a spuit or spotter used in conventional equivalents and thereby enables very minute exposed surfaces of the adsorption/ desorption electrodes to adsorb the molecules, respectively. Namely, the device has an excellent spatial resolution (up to 200 µm). In addition, the device is very advantageous, for example, in the down-sizing, forming into a chip and/or integration of the device, since the exposed surfaces can be further miniaturized by forming the exposed surfaces as a result of patterning the dielectric film with the resist material.

Next, a negative voltage exceeding the threshold is applied to Electrode A to thereby allow Electrode A to desorb the DNA molecule (molecule A) by the action of electrical repulsive force therebetween. Thus, the DNA molecule (molecule A) which has been adsorbed by Electrode A can be retrieved to the outside, by sucking the liquid typically using a pipette or by activating an external electrode applied with a positive voltage. DNA molecules adsorbed by the respective electrodes can be taken out by subjecting the respective electrodes to the above procedure. The retrieved molecules such as DNA molecules may be arranged on, for example, an insulator such as quartz glass. The resulting article is equivalent to a commercially available DNA chip. The retrieved DNA molecules, for example, may be amplified herein. Such commercially available DNA chips are consumed one by one in each test. The device for molecular adsorption or desorption formed into a chip according to the present invention can be repetitively used times corresponding to the number of the adsorption/ desorption electrodes and can be advantageously used in various applications.

A molecule 100 having the target capturer and a light emitting section 110 as shown in Fig. 18 will be illustrated below. The molecule 100 comprises a polynucleotide serving as the interactive region, the light emitting section 110, such as a fluorescent dye, bound to one end of the polynucleotide, and a target capturing section (not shown), such as an antibody, bound to the other end of the polynucleotide.

In Fig. 18, the molecule 100 is adsorbed by the adsorption/desorption electrode, such as a metal electrode, and the light emitting section 110 does not emit light even upon irradiation with light. In Fig. 19, the molecule 100 is desorbed from the adsorption/ desorption electrode, such as a metal electrode, and the light emitting section 110 becomes a light emitting section 110a that emits light upon irradiation with light.

The use of the molecule 100 having the light emitting section 110 can arbitrarily control the light emission thereof by arbitrarily control the timing with which the molecule is desorbed from the adsorption/ desorption electrode, such as a metal electrode. In addition, it can easily detect typically the presence or absence of the target, by detecting a change in the illumination time by utilizing the phenomenon that the diffusion velocity of the molecule 100 itself decreases when the molecule captures the target.

The light irradiating means for applying light to the light emitting section 110 is not specifically limited, can be selected according to the purpose, and suitable examples thereof are ultraviolet lamps and semiconductor laser systems.

The direction of the light applied by the light irradiating means is not specifically limited, can be appropriately selected according to the purpose and is preferably, for example, a direction along the surface of the adsorption/ desorption electrode holding the target capturer and such a direction that the reflected light from the adsorption/ desorption electrode travels toward the light detecting means.

The wavelength of the light applied by the light irradiating means is not specifically limited and can be appropriately set according to the peak wavelength of adsorption by the light emitting section, as long as it is such a wavelength that the light emitting section of the target capturer can adsorb the light and can emit light after absorption.

The light detecting means for detecting light emitted by the light emitting section 110a is not specifically limited and, can be selected according to the purpose, and suitable examples thereof are photoreceivers , CCD cameras, photomultipliers and photodiodes.

The light detecting means is preferably so configured as to determine the number of photons per unit time emitted by the target capturer by calculation; to detect the intensity of fluorescence emitted by the target capturer; or to determine the time between emission and quenching (becoming not detectable) of the light emitted by the target capturer. More preferably, the light detecting means is so configured as to determine the number of photons per unit time emitted by the molecule capturing the target in its target capturing section, which molecule is desorbed from the adsorption-release electrode, and diffuses and migrates from inside a detection region toward outside thereof, to determine the number of photons per unit time emitted by the molecule capturing no target in its target capturing section, which molecule is desorbed from the adsorption-release electrodes, and diffuses and migrates from inside a detection region toward outside thereof, and to compare between the numbers of photons to thereby detect whether or not the target capturing section in the molecule captures the target.

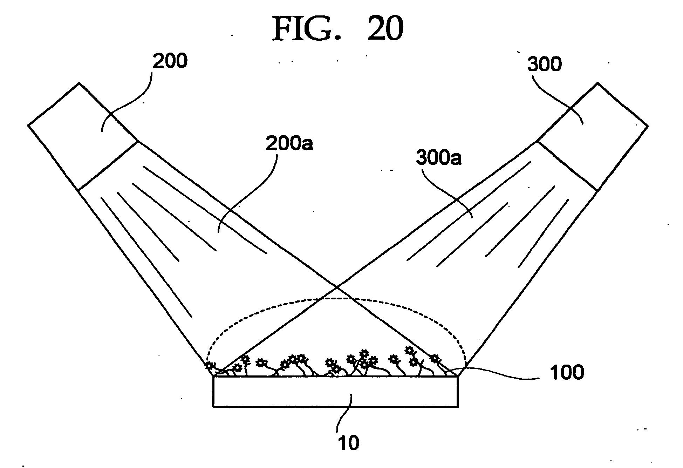

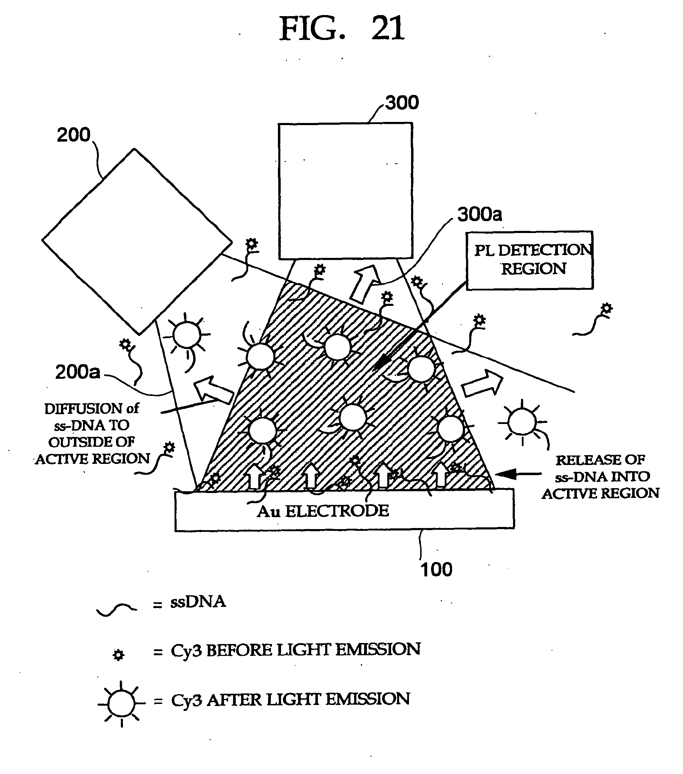

The device for molecular adsorption or desorption having the light emitting section and serving to adsorb or desorb the molecule will be illustrated with reference to Figs. 20 and 21. In the device for molecular adsorption or desorption, DNA molecules 100 each having the target capturing section and the light emitting section are electrically adsorbed by an adsorption/ desorption electrode 10 being applied with a positive voltage. The device also includes an ultraviolet lamp 200 and a photoreceiver 300. The ultraviolet lamp 200 serves as the light irradiating means for applying light to the adsorption/ desorption electrode 10. The photoreceiver 300 serves as the light detecting means.

A counter electrode (not shown) is arranged above the adsorption/desorption electrode 10. The adsorption/ desorption electrode 10 and the counter electrode are connected to a power source (not shown) capable of applying an electric field thereto, are immersed in the electrically conductive liquid and constitute an electric circuit. The electrically conductive liquid contains a sample mixture containing the target which the target capturing section can capture.

The ultraviolet lamp 200 is so arranged that the reflected light by the adsorption/ desorption electrode 10 travels toward the photoreceiver 300. It serves to apply an ultraviolet ray 200a to the surface of the adsorption/ desorption electrode 10 (Figs. 20 and 21). The photoreceiver 300 receives reflected light of the irradiated light 200a and receives light emitted by a light emitting section 110 of the DNA molecule 100 (Figs. 20 and 21).

The DNA molecule 100 is electrically adsorbed by the adsorption/ desorption electrode 10 (Fig. 20), by actuating the power source connected to the adsorption/ desorption electrode 10 to thereby apply a positive voltage to the adsorption/ desorption electrode 10. The light emitting section 110 of the DNA molecule 100 is electrically coupled with the adsorption/ desorption electrode 10 and does not emit light upon irradiation with the ultraviolet ray applied by the ultraviolet lamp 200, and the photoreceiver 300 does not detect light emission. On the other hand, the DNA molecule 100 is desorbed from the adsorption/ desorption electrode 10 by the action of Coulomb repulsive force therebetween (Fig. 21), when an inverse electric potential, i.e., a negative potential, is applied to the adsorption/ desorption electrode to thereby change the potential of the adsorption/desorption electrode 10 to a negative electric potential. The light emitting section 110 of the DNA molecule 100 receives the ultraviolet ray 200a applied by the ultraviolet lamp 200, and the light emitting section 110 absorb the light energy of the ultraviolet ray 200a to thereby emit light (Fig. 21). Accordingly, the photoreceiver 300 detects the light remitted by the light emitting section 110 of the DNA molecule 100.

If the target capturing section of the DNA molecule 100 captures the target under this condition, the DNA molecule 100 shows a decreased molecular diffusion velocity. Thus, the photoreceiver 300 detects an increased quantity of light (number of photons per unit time) emitted by the light emitting section 110 of the DNA molecule 100 as compared with the case where the target capturing section of the DNA molecule 100 is not capturing the target. The photoreceiver 300 can thereby detect the presence or absence of the target, based on the presence or absence of a change in the quantity of emitted light.

The device for molecular adsorption or desorption or the method for molecular adsorption or desorption as described above enables two or more different molecules, such as DNA molecules, to be adsorbed or desorbed arbitrarily and reversibly with different timings, and the device can be miniaturized, formed into a chip and/or integrated. Thus, the device or method can be advantageously used in, for example, diagnosis, analysis, determination and/ or detection of, for example, a variety of diseases including multifactorial diseases such as diabetes mellitus, hypertension and hyperlipidemia. The target capturing section arranged on the molecule enables, for example, the diagnosis, analysis, determination and/or detection of useful molecules such as proteins. In addition, the light emitting section arranged in the molecule enables the quantitative determination of the useful molecules. The device or the method for molecular adsorption or desorption of the present invention allows the molecule to be held or conserved and integrated in an electrically fine space for a short time or long time, can be applied typically to a fine patterning technique in the field of semiconductors in an electrically controllable manner. Thus, the device or method enables adsorption or desorption of a variety of molecules easily and conveniently, is applicable to various researches and contributes to further development of biotechnology industry.

In the following drawings, the same elements may have the same reference numerals. The elements relating to the present invention in the drawings are not always illustrated on an identical scale, and some of them may be largely deformed for the sake of understanding of the present invention.

The device for protein detection achieved by the present invention corresponds to a so-called protein chip. The device aims, for example, to detect the lowering or enhancement of part of an interaction network of a series of proteins from the insulin receptor to glucogenase, in the case where hepatic cells switchover the intracellular glycogen metabolism depending on the condition of receiving insulin in diabetes mellitus.

The present invention enables the grasp of the population of proteins including so-called posttranslational modification such as phosphorylation and glycosylation. The present invention enables the grasp of the phenomenon that, for example, the hypofunction or hypoactivity of a specific protein relating to the interaction network causes insufficient glucose metabolism, which in turn enables an appropriate diagnosis or treatment corresponding to the cause of the malfunction and evaluation of the result of treatment. In contrast, such a phenomenon expressed as a symptom is roughly understood as, for example, diabetes mellitus according to conventional technologies.

The similar technique can also be applied to all the other multifactorial diseases such as hypertension, hyperlipidemia and carcinomata (control failure in cellular proliferation), in addition to diabetes mellitus.

The present invention grasps the presence or absence of a plurality of proteins in a sample, and the type and/or amount (population) of a protein, if present, by arranging, for example, antibodies against proteins to be detected or derivatives thereof in an array and determining the relationship between a signal emitted upon coupling with the target protein and the position in the array.

The present inventors have experimentally observed that, when one end of a single-stranded nucleotide is immobilized to an electrode, is immersed in an aqueous solution, and a direct-current field, for example, is applied to between the electrode and another electrode arranged in the aqueous solution, the strand of the single-stranded nucleotide expands, and when the electric field is turned off, the strand spontaneously contracts. The present invention utilizes this property of nucleotides typified by a single-stranded nucleotide.

Specifically, functions of respective portions of the device for protein detection according to the present invention are as mentioned below.

The first electrode is generally arranged on or above an electrode supporting section having an appropriate shape and plays a role to immobilize the sensing section and a role as part of the control section for changing the conformation, a spatial shape, of the sensing section. The sensing section is generally immobilized to the first electrode at one end of its nucleotide.

The immobilization can be carried out by a known method such as a method in which a nucleotide having a thioether group or a thiol group is synthetically prepared and is brought into contact with a polished surface of the electrode. A bond called as a linker, such as -(CH₂)₃- or -(CH₂)₆-, may be inserted between the thioether group or thiol group and the nucleotide. The number of the sensing section capable of being immobilized to the electrode may often affected by the type of the linker and/ or the length of the bond. The number of the sensing section capable of being immobilized to the electrode generally tends to decrease with a decreasing number of CH₂ units.

The immobilization can also be carried out by using a material called as a carbon nanotube or carbon nanofiber. The carbon nanotube or carbon nanofiber can be prepared, for example, by arranging a catalyst such as Ni on the first electrode and growing a carbon vertically from the surface of the electrode by chemical vapor deposition (CVD) or plasma CVD. The five-membered ring moiety at the tip of the formed carbon nanotube or carbon nanofiber can be easily chemically modified, and a predetermined nucleotide can be bound to the tip of the carbon nanotube or carbon nanofiber using this property. The carbon nanotube or carbon nanofiber is a rigid or hard material, and the nucleotide can be more rigidly bound to the electrode with the interposition of such a rigid material. This configuration is advantageous for example in the use of a viscous sample mixture.

The shape, size, number and arrangement of the electrode can be any according to the purpose. A plurality of proteins can be detected simultaneously, for example, by partitioning the electrode into a plurality of sections using a spacing device or arranging a circular partitioned electrode on an electrode supporting section. In this case, the device must have such functions as to detect fluorescence emission and/or quenching signals from respective partitions separately and distinctively.

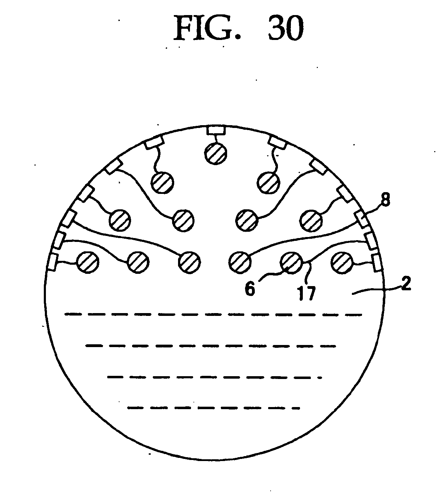

Fig. 30 is a model diagram showing a plurality of circular electrodes 6 are separately arranged on an electrode supporting section 2. Terminals 8 are arranged on the periphery of the electrode supporting section 2 and electrically connected to corresponding electrodes 6 via lead wires 17. The electrodes 6 can be prepared by a photolithographic procedure. The diameter of the electrodes 6 can be about 1 nm to about 1 cm. The electrodes can also have a diameter larger than or smaller than the above-specified range. Electrodes in the lower part of Fig. 30 are not shown. The electrodes 6 are not always to be arranged electrically independently.

The electrode supporting section can be any one that enables the respective electrodes arranged thereon in an insulative status, respectively. Examples thereof are glass, ceramics, plastics and metals. When the electrode supporting section comprises an electrically conductive material, the electrodes may be arranged on the electrode supporting section with the interposition of a dielectric film. For example, when the electrode supporting section comprises Si, a SiO₂ dielectric film may be arranged thereon.

The material for the first electrode can be any electrically conductive material, such as single metals, alloys or layered products thereof. Among them, noble metals typified by Au are chemically stable and are preferably used.

The binding section can comprise at least one selected from the group typically consisting of antibodies capable of binding specifically to the target protein, products as a result of partial hydrolysis of the antibodies with a protease. The binding section is capable of binding specifically to a protein. The device may comprise different materials as the binding sections corresponding to the respective electrodes.

The type and binding site of the bond with the protein are not specifically limited, but a bond having a particularly weak associative strength should preferably be avoided. The term "product" refers to a product obtained by partially hydrolyzing an antibody with a protease and can include, for example, Fab fragments of antibodies, fragments derived from the Fab fragment of antibodies, and derivatives of these substances, as long as they are within the scope of the present invention.

Examples of the antibody are monoclonal immunoglobulin IgG antibodies; Fab fragments of IgG antibodies, as fragments derived from the IgG antibodies; and fragments derived from the Fab fragments. Usable examples of the organic compounds having an affinity for the target protein to be detected or determined are enzyme substrate analogues such as adenosine-5'-O- (3-thiotriphosphate) (synonym: ATP-γ-S); inhibitors of enzyme activity; and neurotransmission inhibitors (antagonists). Examples of the biopolymers having an affinity for the target protein are proteins that serve as a substrate or catalyst for the protein, and constitutional proteins for constituting a molecular complex.

The use of a monoclonal antibody or a product as a result of partial hydrolysis of the antibody with a protease as the binding section is advantageous for utilizing a bond formed as a result of a reaction such as an antigen-antibody reaction.

The binding section preferably comprises a monoclonal antibody, an Fab fragment of a monoclonal antibody, or a fragment derived from the Fab fragment of a monoclonal antibody. The term "fragment derived from the Fab fragment of a monoclonal antibody" means a fragment prepared by dividing the Fab fragment of a monoclonal antibody, or a derivative thereof.

The binding section more preferably comprises an IgG antibody, an Fab fragment of an antibody, or a fragment derived from an IgG antibody or the Fab fragment of an IgG antibody. The term "fragment derived from the Fab fragment of an IgG antibody" means a fragment prepared by dividing the Fab fragment of an IgG antibody, or a derivative thereof. The binding section also preferably comprises a nucleotide aptamer.

These materials are preferred for their high detection sensitivity due to their low molecular weight.

The binding section is bound to a nucleotide strand constituting the sensing section. The type and binding site of the bond with the nucleotide strand are not specifically limited, but a bond having a particularly weak associative strength should preferably be avoided. The binding section is generally preferably bound at the nucleotide strand opposite to the end at which the nucleotide strand is bound to the electrode, since it is generally preferred to expand a polynucleotide to a large extent. If the material constituting the binding section cannot be immobilized directly to the nucleotide strand constituting the sensing section, it may be immobilized with the interposition of an effective lining moiety.

The sensing section comprises the nucleotide strand and a fluorescent dye group, changes its conformation by the action of the control section to allow the fluorescent dye group to emit light or to quench the light to thereby detect the binding of the protein to the binding section. More specifically, a direct-current electric field, for example, is applied to allow the nucleotide strand to expand, and the electric field is turned off to allow the nucleotide strand to contract. This change is a conformational change. An essential feature of the sensing section is to detect the binding of the protein to the binding section. Accordingly, a sensing section not having a nucleotide strand and/or a fluorescent dye group will do, as long as it can detect the binding of the protein to the binding section.

Naturally-occurring nucleotides and artificial nucleotides can be used as the nucleotide for this purpose. The artificial nucleotides include entirely artificial products and derivatives of naturally-occurring nucleotides. The use of an artificial nucleotide may be advantageous for higher detection sensitivity and/or improved stability.

The nucleotide can be a single-stranded nucleotide or a double-stranded nucleotide comprising a pair of complementary single-stranded nucleotides. A single-stranded nucleotide is often preferably used for easy expansion and contraction of the strand. Different nucleotides can be used for the individual electrodes. The length of the nucleotide strand may be at least one residue. Namely, a mononucleotide strand will do.

The fluorescent dye group may be added to the nucleotide strand by the action of a covalent bond, may be contained in the nucleotide strand, such as being inserted or intercalated into between adjacent complementary bonds, or may be incorporated into part of the nucleotide strand as a result of substitution. The fluorescent dye group is preferably arranged in the vicinity of the tip of the nucleotide strand on the binding section side.

The fluorescent dye group is selected from substances or materials that are excited by the action of light to thereby emit fluorescence. Suitable examples of the fluorescent dye group for use in the present invention are fluoroscein maleimide Cy3 (trademark).

The control section plays a role for changing the conformation of the sensing section and includes the first electrode and a second electrode to be inserted into the sample mixture.

Suitable examples of the sample mixture for use in the present invention are aqueous solutions containing proteins. The sample mixture is often used as a buffering solution whose pH has been adjusted.

The device is preferably so configured as to apply an electric field having a constant potential difference or an electric field having a time-variant potential difference to between the first electrode and the second electrode. The device preferably further comprises a standard electrode for compensating the fluctuation in potential difference, in addition to the first electrode and the second electrode. This configuration enables detected signals to be more stable.

The electric field having a constant potential difference can be obtained by using a direct current. The time-variant potential difference can be obtained, for example, the combination of a direct current ingredient, an alternating current ingredient and the presence or absence of a potential difference; an arbitrary combination of pulsed current ingredients whose potential difference varies stepwise; or by modulating such ingredients. The alternating current ingredient herein may have a significantly low wavenumber with a period of 1 second or more. Any materials can be used for the second electrode and the standard electrode, as long as they can yield stable detection results.

The detecting section detects the binding of the protein to the binding section by detecting light emission or quenching by the sensing section. The detecting section can be any known detection device capable of detecting fluorescence and being within the scope of the present invention.

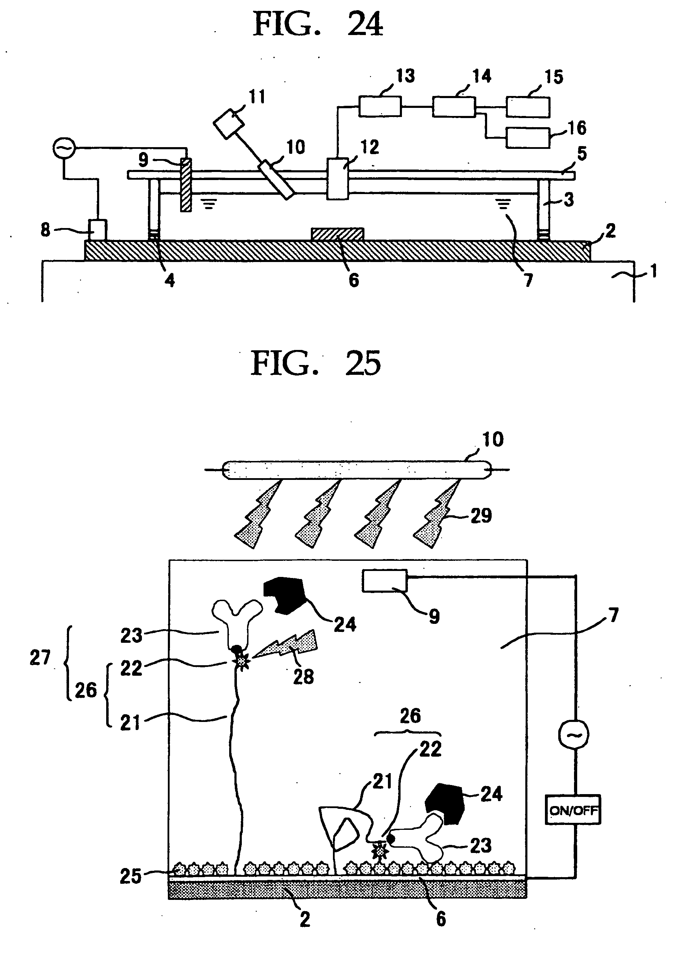

The mechanism of light emission and quenching of the device for protein detection according to the present invention will be illustrated with reference to Figs. 24 and 25. Fig. 24 is a cross sectional view showing an embodiment of the device for protein detection according to the present invention.

With reference to Fig. 24, a sample 7 is placed in a chamber arranged on a base 1. The chamber includes an electrode supporting section 2 and a wall section 3, between which is sealed with an O-ring 4, and the chamber is covered by a lid 5. An electrode 6 is arranged on the electrode supporting section 2 and is connected to an electrode 9 with the interposition of a terminal 8 outside the chamber. The electrode 6 and the electrode 9 correspond to the first electrode and the second electrode, respectively. Plural pieces of the electrode 6 can be arranged. A control section includes the electrode 6 and the electrode 9. The control section can have any configuration within the scope of the present invention.

The device for protein detection having the configuration detects light emission or quenching by the sensing section. The binding section and the sensing section are not shown in the figures.

An excitation light source 10 using a laser oscillator 11, for example, is used. A measuring unit 13 of a sensing element 12 of the device for protein detection is preferably connected to a data processing system 14 for processing measured data. The processing system 14 has a display 15 for measurement results, and a storage device 16 for storing, for example, the array of the electrodes and a calibration value of the detecting unit.

Fig. 25 is a schematic diagram showing emission of fluorescence by the nucleotide strand on the electrode 6 (Fig. 24) upon application of an electric field or quenching of the fluorescence upon removal of the electric field.

The left side of Fig. 25 illustrates a protein detecting unit 27 standing on the electrode 6. The protein detecting unit 27 comprises a binding section 23 and a sensing section 26. The binding section 23 is capable of binding specifically to a protein 24. The sensing section 26 comprises a nucleotide stand 21 and a fluorescent dye group 22 and serves to detect the binding of the protein to the binding section. The protein detecting unit 27 is standing on the electrode 6, because an electric field is applied to the system to allow electrical repulsive force (Coulomb force) to act between the nucleotide strand 21 and the electrode 6 to thereby expand the nucleotide strand 21.

When expanded in this manner, the fluorescent dye group 22 receives little or no electric influence by the electrode 6 and becomes to be able to emit light 28.

The right side of Fig. 25 illustrates a protein detecting unit 27 residing in the vicinity of and over the electrode 6. The protein detecting unit 27 comprises a binding section 23 and a sensing section 26 including a nucleotide strand 21 and a fluorescent dye group 22. This condition is achieved by removal of the electric field from the system, wherein the nucleotide strand 21 becomes an energetically stable structure to thereby aggregate and contract. The nucleotide strand 21 can also contract by actively applying an inverse electric field to apply electrical attractive force (Coulomb force) to between the nucleotide strand 21 and the electrode 6. In the case of a single-stranded nucleotide, the nucleotide can contract without active application of an inverse electric field in many cases, since it has a large molecular flexibility. In the case of a double-stranded nucleotide, an inverse electric field should be preferably actively applied in many cases, since it has a higher molecular rigidity and is often standing (extending vertically) without the application of an electric field.

When the nucleotide strand 21 contracts in this manner, the fluorescent dye group 22 is electrically affected by the electrode 6 to thereby quench the fluorescence. For quenching, the protein detecting unit 27 must not always be in contact with the electrode 6 and may be enough to approach the electrode 6.

Further, the speed and/or efficiency of quenching can be altered by allowing a quencher 25 to bind to the surface of the electrode 6 (Fig. 25). The quencher is selected from among ones that effectively quench the fluorescent dye group to be used. When fluorescein maleimide, Cy3 (trademark), for example, is used as the fluorescent dye group, a quenching dye group such as rhodamine B sulfonyl chloride can be used as the quencher. Such a combination of a fluorescent dye group and a quenching dye group as to yield fluorescence resonance energy transfer (FRET) effect is more preferred.

The excitation light source 10 serving to emit light 29 for exciting the light emitting atomic group can be any of generally used light sources typically of visible rays or ultraviolet lamps.

The light emission and quenching is detected by the sensing element 12 for detecting the emitted light 28. The present invention has been illustrated by taking, as an example, the above-mentioned expansion and contraction behavior. However, the case where quenching occurs when the nucleotide strand 21 expands and light emission occurs when the nucleotide strand 21 contracts is also included within the scope of the present invention. In this configuration, a quencher is not used, but a light emission auxiliary for promoting light emission can be used.

Regarding the relationship between the application or removal of the electric field and the expansion/contraction behavior of the nucleotide strand, the nucleotide strand can expand or contract depending on the electric field, if applied. In contrast, the nucleotide strand can contract upon removal of the electric field.

Regarding the relationship between the application or removal of an electric field and the emission or quenching of fluorescence, there are the cases where light emission occurs upon the application of an electric field, where quenching occurs upon application of an electric field, where light emission occurs upon removal of an electric field, and where quenching occurs upon removal of an electric field. The phase "nucleotide strand in an initial state emits or quench fluorescence upon the application or removal of an electric field" as used herein means and includes all the above-mentioned cases.

The use of this device enables detection of the presence or absence of the binding of a protein to the binding section, the type and/ or amount of the bound protein by detecting a change in light emission or quenching in the case where the nucleotide strand in an initial state emits or quench fluorescence upon application or removal of an electric field.

In the right and left sides of Fig. 25, the protein 24 is bound to the binding section 23. When the protein 24 is bound to the binding section 23, it takes a longer time for the nucleotide strand 21 to expand and contract than in the case where the protein 24 is not bound to the binding section 23. Accordingly, it takes a longer time for the light emission and quenching to change. The "initial state before the application of an electric field" herein means a status in which no electric field is applied or a status in which an electric field to form an inverse potential difference is applied. The "initial state before removal of an electric field" means a status in which an electric field is applied.

Initially, the presence of binding of a protein to a binding section can be detected by detecting a change in light emission or quenching where fluorescence is emitted or quenched upon the application or removal of an electric field. The type of the protein can be determined by determining the used binding section is capable of binding to which kind of proteins. This detection can be easily carried out, since the binding section relating to the present invention is capable of binding to a specific protein.

Next, the amount of the protein bound to the binding section can be determined by determining a change in light emission or quenching.

More specifically, the amount can be determined, for example, based on the intensity or the rate of change of emission in the emission or quenching of fluorescence.

The amount can also be determined based on the peak intensity of emitted fluorescence, the change thereof or the rate of change thereof upon the application of an electric field having a time-variant potential difference.

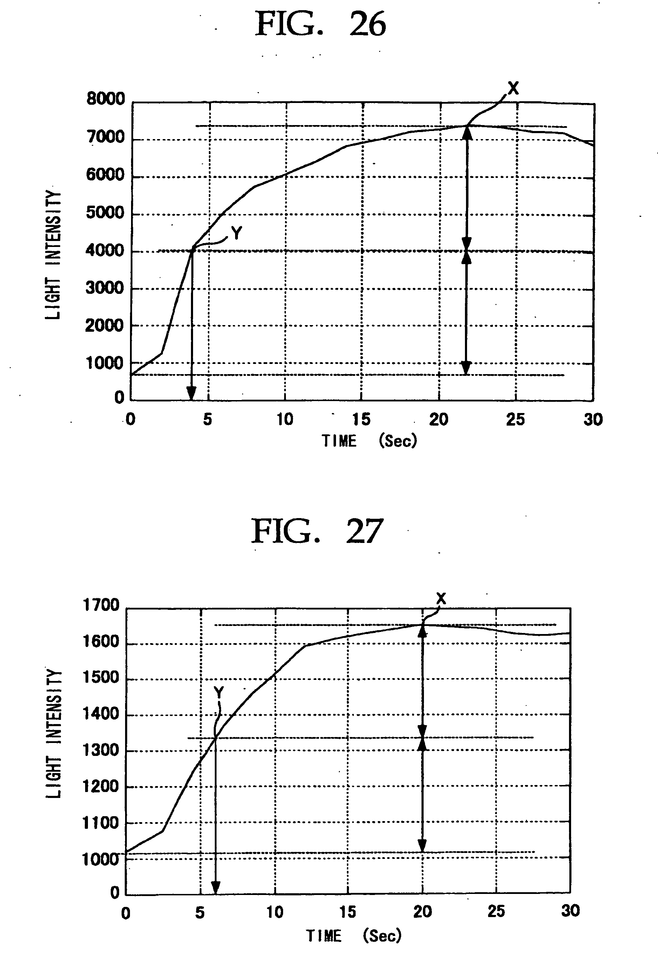

Figs. 26 and 27 are diagrams showing a change of emission intensity with time in the cases where no protein is bound and where a protein is bound, respectively, with the abscissa showing time from the initial of application of an electric field. The unit thereof can be arbitrarily set, but Figs. 26 and 27 employ a common unit.

In Figs. 26 and 27, the time taken for the emission intensity to reach Point Y is taken as a specific example of change in light emission. At Point Y, the emission intensity is half of the fluorescence emitting peak at Point X. Comparison of Fig. 27 with Fig. 26 shows that the time is longer in Fig. 27 than in Fig. 26. It takes a shorter time between Point Y and Point X in Fig. 27 than in Fig. 26, because the peak value is much smaller in Fig. 27 than in Fig. 26. Namely, it takes a longer time for the emission intensity to reach the substantially same value in Fig. 27 than in Fig. 26.

The time to reach Point Y is probably affected typically by the type, size and charge of a protein to be detected. When the protein has a large size, it takes a longer time for the nucleotide to expand due to its mass effect and/or shape effect.

In addition, the time also depends on the amount of the protein bound to the binding section. This is probably because the binding between the protein and the binding section makes protein detecting units on the electrode crowded or packed to thereby inhibit the expansion of the nucleotide.

The emission intensity upon fluorescence emission includes, for example, an emission intensity (peak value) at Point X. The rate of change in emission intensity includes, for example, an average of rate of change in light emission from the beginning of light emission to Point Y, and an average of rate of change in light emission from Point Y to Point X.

Comparison between Fig. 27 and Fig. 26 shows that the average of rate of change in light emission from the beginning of light emission to Point Y, and the average of rate of change in light emission from Point Y to Point X are apparently lower in Fig. 27. This is probably because the nucleotide becomes resistant to expansion, since the nucleotide moves with an increased resistance to the solvent due to binding between its binding section and the protein.

The results shown in Figs. 26 and 27 are obtained by applying an electric field with a constant potential difference or a time-variant potential difference to between the first electrode and the second electrode.

When an electric field having a time-variant potential difference, such as an alternating current, is applied, the potential difference varies with time or the direction of the electric field changes with time, and thereby the light emission repetitively increases and decreases.

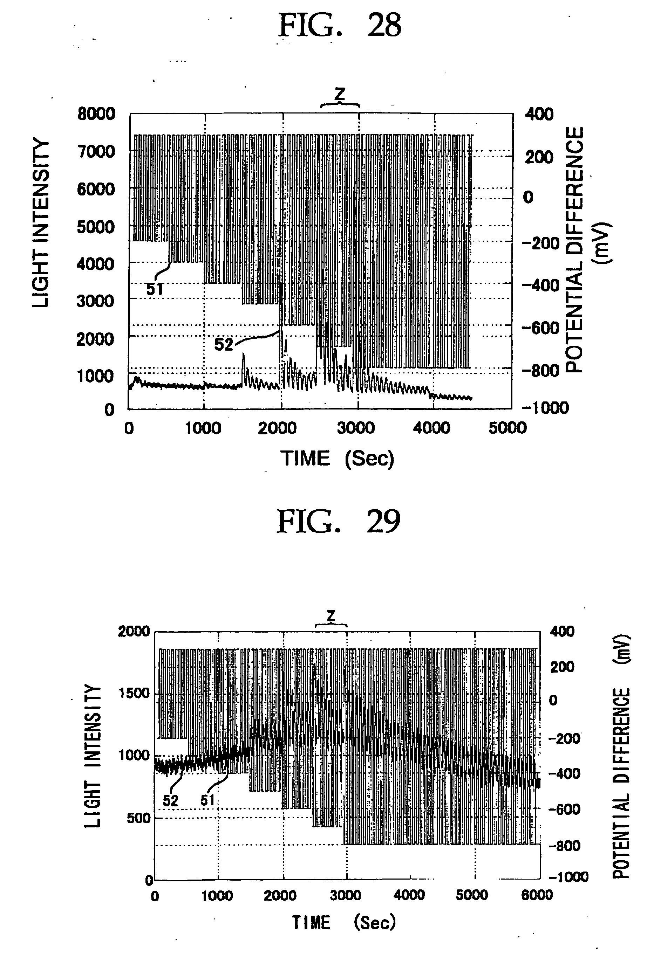

Figs. 28 and 29 are diagrams showing changes of emission intensity with time upon application of a voltage having a pulse waveform, in the cases where no protein is bound and where a protein is bound, respectively.

In Figs. 28 and 29, the abscissa represents the elapse of time upon application of a certain electric field. The right ordinate represents the potential difference between the first electrode and the second electrode. An applied potential difference 51 has a rectangular pulse shape. The left ordinate represents the emission intensity. The unit of the emission intensity is arbitrarily set, but a common unit is used in Figs. 28 and 29. The emission intensity 52 varies in a sawtooth shape.

Figs. 28 and 29 show that the peak value decreases when a protein is bound even when an electric field having a waveform with the same potential difference is applied, and that the peak value decreases in a different manner as shown by region Z even when the waveform with the same potential difference is maintained.

In generalities, when an electric field with a time-variant potential difference is applied, the peak intensity and/or rate of change thereof varies depending on the presence or absence of the binding of a protein, as well as depending on the type and/or amount of the bound protein. The electric field with a time-variant potential difference can be obtained by arbitrarily combining or modulating an alternating current ingredient and/or a pulsed current ingredient.

Based on the above-mentioned variation or change, the , presence or absence of the binding of a protein to the binding section, the type and/or amount of the bound protein can be detected.

Enlarged views of portions where the emission intensity increases in Figs. 28 and 29 show similar curves to those in Figs. 26 and 27, respectively.

As is described above, the present invention serves to detect and determine the presence or absence, the type and amount of a protein easily and conveniently. In addition, the present invention does not require labeling to the protein.

The present invention will be illustrated in further detail with reference to several examples below, which are never intended to limit the scope of the present invention.

### (Example 1)

Initially, a single-stranded polynucleotide having twelve bases and containing (CH₂)₃ SS (CH₂)₃ OH at the three prime-end and the fluorescent dye represented by Structural Formula 1 and biotin at the five prime-end was synthetically prepared as the target capturer. In the target capturer, the fluorescent dye serves as the light emitting section, the biotin serves as the target capturing section, and the polynucleotide serves as the interacting section.

The target capturer was allowed to react with a polished circular metal electrode 1 (a gold electrode in this example) having a diameter of 7 mm at room temperature for 24 hours. The metal electrode 1 and a reference electrode facing the metal electrode 1 were placed in an electrolytic solution, a direct-current electric field was applied to between the two electrodes to thereby apply a positive electric field to the metal electrode 1 (gold electrode). Then, the single-stranded nucleotide serving as the interacting section of the target capturer electrically interacted (bound) with the metal electrode 1 (gold electrode) (Fig. 5). The number of the target capturer electrically coupled with the metal electrode 1 (gold electrode) was determined by calculation with reference to J. Am. Chem. Soc. 1999, 121,10803-10821 and was found to be 1.8×10¹⁰. Under this condition, an ultraviolet lamp 2 applies an ultraviolet ray 2a to the target capturer on the metal electrode 1, and the fluorescent dye of the target capturer did not emit fluorescence.

Next, a direct-current electric field was applied to between the two electrodes to thereby apply a negative electric field modulated as, for example, being pulsed to the metal electrode 1 (gold electrode). Then, with reference to Fig. 6, the target capturer which had not been rigidly bound to the metal electrode 1 with the interposition of SS bond was released from the metal electrode 1 (gold electrode) by the action of Coulomb repulsive force (Fig. 5). Under this condition, the target capturer released from the metal electrode 1 was applied with the ultraviolet ray 2a from the ultraviolet lamp 2, and the fluorescent dye serving as the light emitting section was excited and emitted fluorescence. A photoreceiver 3 detected the light emission by the fluorescent dye.

When the electrolytic solution did not contain avidin, a protein capable of binding to biotin in the target capturer, the target capturer diffused in the electrolytic solution at a high velocity, and the number of photons per unit time of the fluorescence emitted by the fluorescent dye in the target capturer decreased to a large extent (large fluctuation in fluorescence intensity) (Fig. 7).

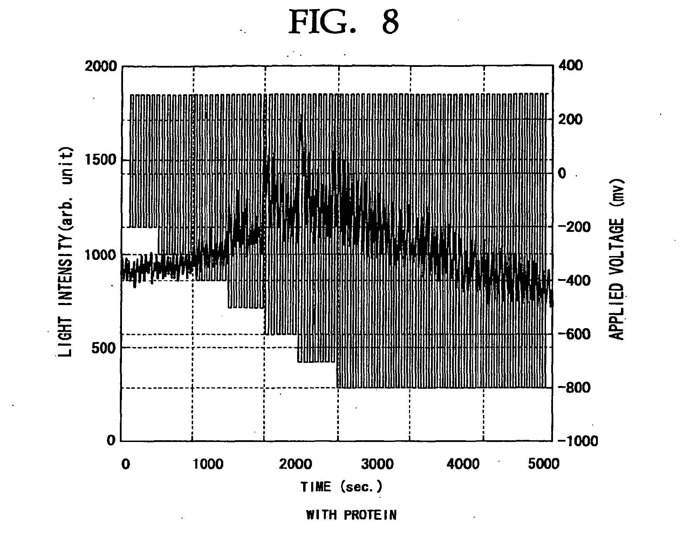

In contrast, when the electrolytic solution contained avidin, a protein capable of binding to biotin in the target capturer, the target capturer was bound to avidin and thereby diffused in the electrolytic solution at a low velocity, and the number of photons per unit time of the fluorescence emitted by the fluorescent dye in the target capturer decreased to a small extent (small fluctuation in fluorescence intensity) (Fig. 8).

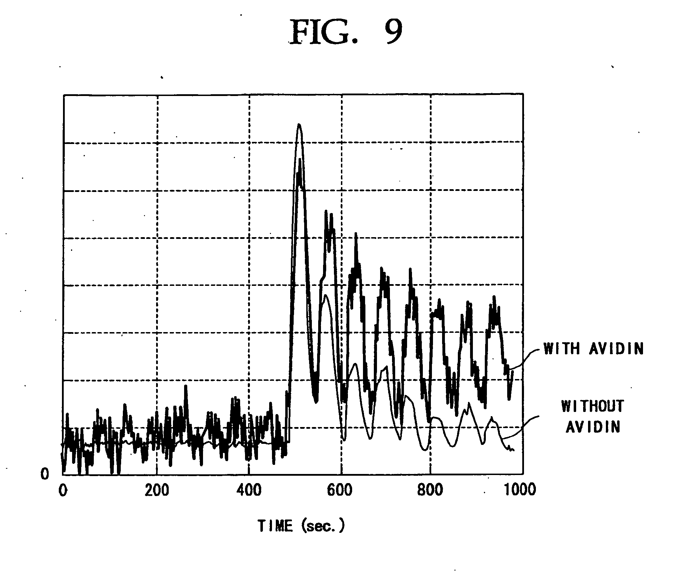

Fig. 9 is a combined view of rates of decrease in number of photons of fluorescence emitted by the fluorescent dye of the target capturer per unit time in the presence of or in the absence of avidin in the electrolytic solution. Fig. 9 shows that the respective peaks decay to a less extent in the presence of avidin in the electrolytic solution than in the absence thereof.

### (Example 2)

An example of the device or method for molecular adsorption or desorption or of the present invention is as mentioned above. A concrete test on the adsorption and desorption of the molecule in the adsorption/ desorption electrode will be illustrated below.

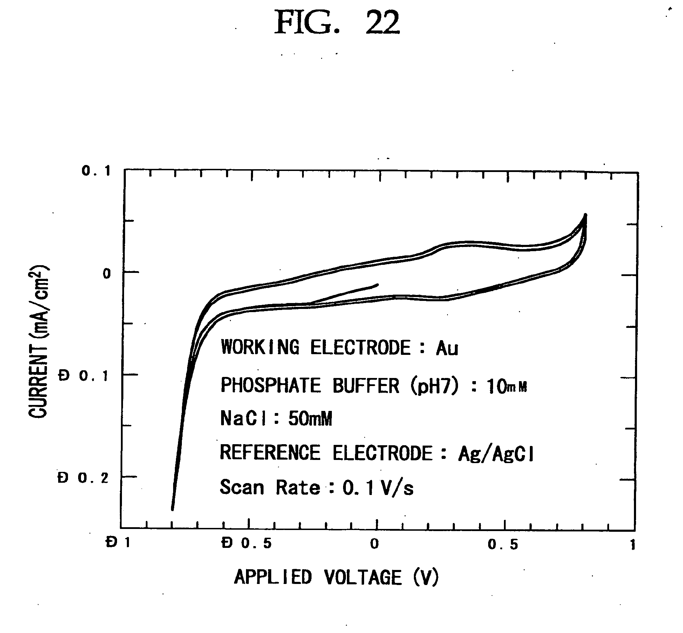

A gold electrode serving as the adsorption/ desorption electrode was formed on a quartz glass substrate as the substrate by vapor deposition. A dielectric film of silicon nitride was formed on the surface of the substrate and the gold electrode to expose part of the substrate and the gold electrode therefrom (Figs. 10A and 10B). The gold electrode was evaluated by cyclic voltammetry, an electrochemical measuring process, and the result is shown in Fig. 22. The graph shows that the gold electrode has a low current at voltages from -0.7 V to 0.7 V, indicating that there is no significant oxidation-reduction reaction on the surface thereof, indicating that the gold electrode can be used without problems such as breakage in the application of a voltage from -0.7 V (-700 mV) to 0.7 V (700 mV).

Next, the adsorption/desorption electrode 10 (gold electrode) was immersed in a 0.05 mol/L aqueous sodium chloride (NaCl) solution, and a positive voltage (700 mV) was applied thereto (Fig. 13). A ss-DNA (single-stranded DNA) serving as the molecule was added to the aqueous NaCl solution. Then, the ss-DNA which was strongly negatively charged was adsorbed by the gold electrode to which the positive voltage was applied.

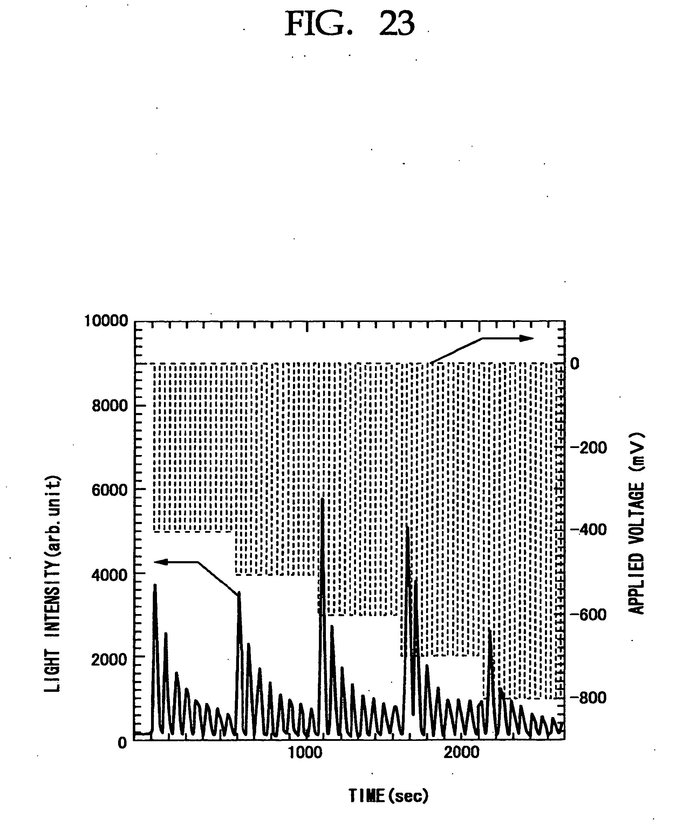

Next , a reference electrode 14(Ag/AgCl) was inserted into the aqueous NaCl solution, and a voltage varying from -600 mV to -800 mV was applied to the gold electrode adsorbing the ss-DNA using the reference electrodes 14 as a standard (Fig. 14). Then, the ss-DNA was desorbed into the aqueous NaCl solution. In this procedure, a dye (Cy3) serving as the light emitting section had been bound to the ss-DNA. The dye did not emit light emission when the ss-DNA was adsorbed by the gold electrode, and emitted light when the ss-DNA was desorbed from the gold electrode and migrated into the aqueous NaCl solution. Fig. 23 is a graph showing the light emission in this test.

### (Example 3)

The device shown in Figs. 24 and 25 was used herein.

Single-stranded oligonucleotides having a thiol group with the interposition of a spacer at the three prime-end were synthetically prepared by using a naturally-occurring single-stranded oligonucleotide and an artificial single-stranded oligonucleotide and were allowed to react with a polished gold electrode at room temperature for 24 hours reaction. Thus, the naturally-occurring single-stranded oligonucleotide and the artificial single-stranded oligonucleotide were allowed to bind with the gold electrode arranged on sapphire (Fig. 24). A fluorescent dye group had been introduced into the single-stranded oligonucleotides. The thiol group and the fluorescent dye group may be introduced at the end of the single stranded or at the five prime-end of the strand.

The oligonucleotide strands were immobilized to the circular gold electrode having a diameter of 1 mm. No quencher was used in the present example.

Fab fragments of monoclonal immunoglobulin IgG were immobilized to terminals of the oligonucleotide strands. In this procedure, Fab fragments having different specificities were immobilized to different oligonucleotide groups partitioned by a spacing device.

A sample solution containing a protein to be detected was brought into contact with the device and was left at room temperature for ten minutes, a sufficient time for the detecting section and the protein to combine with each other.

The electrode after the above treatment bearing the oligonucleotide strands was immersed in an aqueous solution, an electric field of direct current or alternating current was applied to the oligonucleotide strands, and the fluorescent dye group of the oligonucleotide strands was excited by application of light from the ultraviolet lamp to emit fluorescence. The fluorescence intensity varying with time was determined. This procedure was carried out according to a two-electrodes process using the first electrode and the second electrode, and according to a three-electrodes process using the first electrode, the second electrode and a standard (reference) electrode. A more stable result was obtained in the three-electrodes process.

Figs. 26 to 29 show the results of this test.

When an electric field was applied so as to apply a negative electric potential to the first electrode, the oligonucleotide behaving as a negative ion expanded by the action of Coulomb repulsion, and the fluorescent dye group bound to the oligonucleotide dissociated itself from the electrode. As a result, the fluorescent dye group began to emit light, which fluorescent dye group had quenched because of being in the vicinity of the first electrode.

When the sample solution contained the protein to be detected, the Fab fragment was bound to the protein to increase the mass or size of a tip part of the single-stranded oligonucleotide, and it took a longer time for the fluorescence intensity to increase.

When the electric field was removed or an electric field was applied so as apply a negative electric potential to the first electrode, the oligonucleotide strand contracted spontaneously or by the action of Coulomb force. As a result, the fluorescent dye group approached to the surface of the electrode to thereby decrease the fluorescence intensity.

In this procedure, when the sample solution contained a protein to be detected, it took a longer time for the fluorescence intensity to decrease, as in the case where an electric field was applied so as to apply a negative electric potential to the first electrode. The degree of increase of the time varies depending on the amount of the protein in the sample, and by using this, the amount of the protein in the sample could be determined. In addition, the type of the protein could be identified by determining which oligonucleotide strand emitted the signal.

When an electric field is applied so as to apply a negative electric potential to the first electrode, the fluorescence intensity increases. The test results show that the emission intensity increases to a higher extent when no protein is bound to the oligonucleotide, since the single-stranded oligonucleotide sufficiently expands to as to increase the distance between the electrode and the fluorescent dye group, and that the fluorescence intensity does not increase so much when a protein is bound to the oligonucleotide, since the single-stranded oligonucleotide does not sufficiently expand due to the presence of the protein.

Figs. 28 and 29 show that the peak value decreases with time even when the waveform of the same potential difference is maintained as in the region Z. This behavior also contributes to the determination of the amount and/or type of the protein in the sample, as described above.

Figs. 28 and 29 show an example in which a pulse voltage was applied. Even if containing an alternating current ingredient, the data on the emission intensity can contribute to the determination of the amount and/or type of the protein in the sample.

The degree of increase of the time varies depending on the amount of the protein in the sample, and by using this, the amount of the protein in the sample could be determined. In addition, the type of.the protein could be identified by determining which oligonucleotide strand emitted the signal.

The detection sensitivity of a protein varies depending on the molecular weight (size) of the protein to be coupled and on the coupling constant between the protein and, for example, a monoclonal IgG antibody. Specifically, the determination can be carried out within a wide range, for example, by arranging a plurality of monoclonal antibodies having different coupling constants in an array.

Preferred aspects of the present invention are as follows. (Aspect A-1) A target capturer comprising an interacting section, a target capturing section and a light emitting section, the interacting section at least partially containing a region interactive with an electrically conductive member, the target capturing section capable of capturing a target, and the light emitting section capable of emitting light upon irradiation with light when the region in the interacting section does not interact with the electrically conductive member. (Aspect A-2) A target capturer according to Aspect A-1, wherein the interacting section is linear or filamentous.
(Aspect A-3) A target capturer according to one of Aspects A-1 and A-2, wherein the interacting section comprises an ionic polymer. (Aspect A-4) A target capturer according to Aspect A-3, wherein the ionic polymer is selected from among cationic polymers and anionic polymers.
(Aspect A-5) A target capturer according to one of Aspects A-3 and A-4, wherein the ionic polymer is a polynucleotide.
(Aspect A-6) A target capturer according to Aspect A-5, wherein the polynucleotide is selected from among DNAs and RNAs.
(Aspect A-7) A target capturer according to one of Aspects A-5 and A-6, wherein the polynucleotide comprises at least six bases.
(Aspect A-8) A target capturer according to any one of Aspects A-1 to A-7, wherein the target capturing section is selected from among antibodies, antigens, enzymes and coenzymes.
(Aspect A-9) A target capturer according to any one of Aspects A-1 to A-8, wherein the light emitting section comprises a fluorescent dye. (Aspect A-10) A target capturer according to any one of Aspects A-1 to A-9, wherein the target is an organic molecule.
(Aspect A-11) A target capturer according to Aspect A-10, wherein the organic molecule is selected from among proteins, lipoproteins, glycoproteins, polypeptides, lipids, polysaccharides, lipopolysaccharides, nucleic acids and medicaments.
(Aspect A-12). A target capturer according to any one of Aspects A-1 to A-11, wherein the interaction with the electrically conductive member is an electric interaction.
(Aspect A-13) A target detecting device comprising a target capturer, means for releasing the target capturer, light irradiating means and light detecting means, the target capturer at least partially containing a region interactive with an electrically conductive member, being capable of capturing a target, and being capable of emitting light upon irradiation with light in the case of not interacting with the electrically conductive member, the means for releasing the target capturer serving to release the target capturer from the electrically conductive member by ceasing the interaction between the target capturer and the electrically conductive member, the light irradiating means serving to apply light to the electrically conductive member, and the light detecting means serving to detect light emitted by the target capturer upon irradiation of light applied by the light irradiating means. (Aspect A-14) A target detecting device according to Aspect A-13, wherein the target capturer comprises an interacting section, a target capturing section and a light emitting section, wherein the interacting section at least partially contains a region interactive with the electrically conductive member, wherein the target capturing section is capable of binding to a target, wherein the light emitting section is capable of emitting light upon irradiation with light in the case where the region in the target capturing section is not interacting with the electrically conductive member.
(Aspect A-15) A target detecting device according to Aspect A-14, wherein the interacting section is linear or filamentous.
(Aspect A-16) A target detecting device according to one of Aspects A-14 and A-15, wherein the interacting section comprises an ionic polymer.
(Aspect A-17) A target detecting device according to Aspect A-16, wherein the ionic polymer is selected from among cationic polymers and anionic polymers.
(Aspect A-18) A target detecting device according to one of Aspects A-16 and A-17, wherein the ionic polymer is a polynucleotide.
(Aspect A-19) A target detecting device according to Aspect A-18, wherein the polynucleotide is selected from among DNAs and RNAs.
(Aspect A-20) A target detecting device according to one of Aspects A-18 and A-19, wherein the polynucleotide comprises at least six bases.
(Aspect A-21) A target detecting device according to any one of Aspects A-14 to A-20, wherein the target capturing section is selected from among antibodies, antigens, enzymes and coenzymes.
(Aspect A-22) A target detecting device according to any one of Aspects A-14 to A-21, wherein the light emitting section comprises a fluorescent dye.
(Aspect A-23) A target detecting device according to any one of Aspects A-13 to A-22, wherein the target is an organic molecule.
(Aspect A-24) A target detecting device according to Aspect A-23, wherein the organic molecule is selected from among proteins, lipoproteins, glycoproteins, polypeptides, lipids, polysaccharides, lipopolysaccharides, nucleic acids and medicaments.
(Aspect A-25) A target detecting device according to any one of Aspects A-13 to A-24, wherein the interaction with the electrically conductive member is an electric interaction.
(Aspect A-26) A target detecting device according to any one of Aspects A-13 to A-25, wherein the electrically conductive member is one or more electrodes, and wherein the device further comprises one or more reference electrodes arranged so as to face the electrically conductive member.
(Aspect A-27) A target detecting device according to Aspect A-26, further comprising a standard electrode.
(Aspect A-28) A target detecting device according to any one of Aspects A-13 to A-27, wherein the means for releasing the target capturer is so configured as to apply an inverse electric field to the electrically conductive member to thereby release the target capturer from the electrically conductive member by the action of electrical repulsive force, the target capturer having interacted with the electrically conductive member as a result of application of an electric field to the electrically conductive member, and the inverse electric field being opposite to the electric field.
(Aspect A-29) A target detecting device according to any one of Aspects A-13 to A-27, wherein the means for releasing the target capturer is so configured as to apply a stimulus to the target capturer to cleave part of the target capturer to thereby release the target capturer from the electrically conductive member, the target capturer having interacted with the electrically conductive member.
(Aspect A-30) A target detecting device according to Aspect A-29, wherein the stimulus is selected from among light, electricity, agents and enzymes.
(Aspect A-31) A target detecting device according to any one of Aspects A-13 to A-30, wherein the light irradiating means is an ultraviolet lamp.
(Aspect A-32) A target detecting device according to any one of Aspects A-13 to A-31, wherein the light detecting means is so configured as to receive light reflected by the electrically conductive member and to detect light from a light source.
(Aspect A-33) A target detecting device according to any one of Aspects A-13 to A-32, wherein the light detecting means is so configured as to determine the number of photons emitted by the target capturer per unit time.
(Aspect A-34) A target detecting device according to any one of Aspects A-13 to A-33, wherein the light detecting means is so configured as to determine the number of photons per unit time emitted by a target capturer capturing the target, which target capturer is released from the electrically conductive member and diffuses and migrates from inside a detection region toward outside thereof, to determine the number of photons per unit time emitted by a target capturer capturing no target, which target capturer is released from the electrically conductive member and diffuses and migrates from inside a detection region toward outside thereof, and to compare between the numbers of photons to thereby detect whether or not the target capturer captures the target.
(Aspect A-35) A target detecting device according to any one of Aspects A-13 to A-34, wherein the electrically conductive member is an electrode substrate.

The present invention can solve the problems in conventional technologies and provide a target detecting device capable of efficiently detecting a variety of targets such as proteins without labeling typically with fluorescence, and a target capturer advantageously usable in the device.
(Aspect B-1) A device for molecular adsorption or desorption, comprising two or more adsorption/desorption electrodes capable of being independently controlled and carrying out one of adsorption and desorption of a molecule.
(Aspect B-2) A device for molecular adsorption or desorption according to Aspect B-1, wherein the two or more adsorption/desorption electrodes reversibly carry out adsorption and desorption of the molecule.
(Aspect B-3) A device for molecular adsorption or desorption according to one of Aspects B-1 and B-2, wherein the two or more adsorption/desorption electrodes adsorb and/or desorb different molecules, respectively.
(Aspect B-4) A device for molecular adsorption or desorption according to any one of Aspects B-1 to B-3, wherein the two or more adsorption/desorption electrodes are arranged on or above the same substrate.
(Aspect B-5) A device for molecular adsorption or desorption according to any one of Aspects B-1 to B-4, further comprising at least one counter electrode constituting an electric circuit with the two or more adsorption/desorption electrodes.
(Aspect B-6) A device for molecular adsorption or desorption according to Aspect B-5, wherein the two or more adsorption/desorption electrodes and the counter electrode are arranged on or above the same substrate.
(Aspect B-7) A device for molecular adsorption or desorption according to any one of Aspects B-5 and B-6, wherein the device comprises one counter electrode, and the two or more adsorption/desorption electrodes are arranged so as to face the counter electrode.
(Aspect B-8) A device for molecular adsorption or desorption according to any one of Aspects B-5 to B-7, further comprising at least one reference electrode.
(Aspect B-9) A device for molecular adsorption or desorption according to Aspect B-8, wherein the two or more adsorption/ desorption electrodes, the at least one counter electrode and the at least one reference electrode are arranged on or above the same substrate.
(Aspect B-10) A device for molecular adsorption or desorption according to one of Aspects B-8 and B-9, wherein the device comprises one counter electrode and two reference electrodes, wherein the two electrodes are arranged so as to sandwich the counter electrode, and wherein the two or more adsorption/ desorption electrodes are arranged so as to sandwich the counter electrode and the reference electrodes.
(Aspect B-11) A device for molecular adsorption or desorption according to any one of Aspects B-4 to B-10, comprising two or more substrates.
(Aspect B-12) A device for molecular adsorption or desorption according to Aspect B-11, wherein each of the substrates has a total of n adsorption/ desorption electrodes comprising first, second,....... (n-1)th and (n)th adsorption/desorption electrodes, and wherein the first adsorption/desorption electrodes in the respective substrates are electrically connected to an identical power source, the second adsorption/desorption electrodes are electrically connected to an identical power source,....... the (n-1)th adsorption/desorption electrodes are electrically connected to an identical power source, and the (n)th adsorption/desorption electrodes are electrically connected to an identical power source, respectively.
(Aspect B-13) A device for molecular adsorption or desorption according to Aspect B-12, wherein the first adsorption/desorption electrodes, the second adsorption/desorption electrodes, ......the (n-1)th adsorption/desorption electrodes and the (n)th adsorption/desorption electrodes in adjacent substrates face each other, respectively.
(Aspect B-14) A device for molecular adsorption or desorption according to any one of Aspects B-1 to B-13, wherein at least one of the two or more adsorption/ desorption electrodes is coated with a dielectric film so that part of the at least one adsorption/ desorption electrode is exposed from the dielectric film.
(Aspect B-15) A device for molecular adsorption or desorption according to Aspect B-14, wherein the exposed area of the adsorption/desorption electrode has a substantially rectangular shape with a width of 500 µm or less.
(Aspect B-16) A device for molecular adsorption or desorption according to one of Aspects B-14 and B-15, wherein the dielectric film is a patterned resist film.
(Aspect B-17) A device for molecular adsorption or desorption according to Aspect B-16, wherein the resist film is formed from at least one selected from g-line resists, i-line resists, KrF resists, ArF resists, F2 resists and electron beam resists.
(Aspect B-18) A device for molecular adsorption or desorption according to any one of Aspects B-1 to B-17, wherein the interval between the exposed areas in the adjacent adsorption/desorption electrodes of the two or more adsorption/ desorption electrodes is 100 nm or more.
(Aspect B-19) A device for molecular adsorption or desorption according to any one of Aspects B-1 to B-18, comprising an insulating substrate.
(Aspect B-20) A device for molecular adsorption or desorption according to any one of Aspects B-1 to B-19, further comprising an adhesion layer between a substrate and the adsorption/desorption electrodes, the adhesion layer serving to bring the adsorption/desorption electrodes and the substrate into intimate contact with each other.
(Aspect B-21) A device for molecular adsorption or desorption according to any one of Aspects B-1 to B-20, wherein the molecule at least partially comprises an electrically interactive region.
(Aspect B-22) A device for molecular adsorption or desorption according to any one of Aspects B-1 to B-21, wherein the molecule is a linear or filamentous molecule.
(Aspect B-23) A device for molecular adsorption or desorption according to any one of Aspects B-1 to B-22, wherein the molecule is a biomolecule.
(Aspect B-24) A device for molecular adsorption or desorption according to any one of Aspects B-1 to B-23, wherein the molecule is a charged molecule.
(Aspect B-25) A device for molecular adsorption or desorption according to any one of Aspects B-1 to B-24, wherein the molecule has a target capturing section capable of capturing a target.
(Aspect B-26) A device for molecular adsorption or desorption according to Aspect B-25, wherein the target capturing section is selected from among antibodies, antigens, enzymes and coenzymes.
(Aspect B-27) A device for molecular adsorption or desorption according to any one of Aspects B-1 to B-26, wherein the molecule has a light emitting section capable of emitting light upon irradiation with light in the case of not interacting with the adsorption/ desorption electrode.
(Aspect B-28) A device for molecular adsorption or desorption according to Aspect B-27, wherein the light emitting section comprises a fluorescent dye.
(Aspect B-29) A device for molecular adsorption or desorption according to any one of Aspects B-1 to B-28, wherein the adsorption/desorption electrodes are immersed in an electrically conductive liquid containing the molecule upon use.
(Aspect B-30) A method for molecular adsorption or desorption, comprising the step of applying electric potentials to two or more adsorption/desorption electrodes, the electric potentials arbitrarily varying with different timings, to thereby allow the two or more adsorption/ desorption electrodes to carry out one of adsorption and desorption of a molecule with different timings.
(Aspect B-31) A method for molecular adsorption or desorption according to Aspect B-30, wherein the two or more adsorption-release electrodes adsorb or desorb different molecules, respectively.
(Aspect B-32) A method for molecular adsorption or desorption according to one of Aspects B-30 and B-31, further comprising carrying out the molecular adsorption and desorption reversibly.
(Aspect B-33) A method for molecular adsorption or desorption according to any one of Aspects B-30 to B-32, further comprising applying a lubricant to the surfaces of the adsorption/ desorption electrodes before adsorption or desorption of the molecule.
(Aspect B-34) A method for molecular adsorption or desorption according to any one of Aspects B-30 to B-33, further comprising immersing the adsorption/desorption electrodes in an electrically conductive liquid containing the molecule.

The present invention can meet the demands, solve the problems in conventional technologies and provide a device for molecular adsorption or desorption which is capable of efficiently and reliably adsorbing and/ or desorbing one or more useful substances or molecules, such as DNAs, with different timings arbitrarily, is capable of down-sized, formed into chips and/or integrated, is suitable typically for gene therapy, diagnosis and/or analysis and is safe. In addition, the present invention can provide a method for molecular adsorption or desorption which is capable of efficiently and reliably adsorbing or desorbing one or more useful substances or molecules, such as DNAs, with different timings arbitrarily, is suitable typically for diagnosis and/or analysis and is safe.
(Aspect C-1) A device for protein detection, comprising:
a binding section capable of binding specifically to a protein;
a sensing section for detecting the binding of the protein to the binding section;
a first electrode serving to immobilize the sensing section;
a second electrode;
a control section for controlling the conformation of the sensing section, the control section including the first electrode and the second electrode; and
a detecting section for detecting emission or quenching of light by the sensing section.
(Aspect C-2) A device for protein detection, comprising:
a binding section capable of binding specifically to a protein;
a sensing section for detecting the binding of the protein to the binding section, the sensing section comprising a nucleotide strand and a fluorescent dye group;
a first electrode serving to immobilize the sensing section;
a second electrode;
a control section for controlling the conformation of the sensing section, the control section including the first electrode and the second electrode; and
a detecting section for detecting emission or quenching of light by the sensing section.
(Aspect C-3) A device for protein detection according to any one of Aspects C-1 and C-2, wherein the control section further comprises a reference electrode.
(Aspect C-4) A device for protein detection according to any one of Aspects C-1 to C-3, wherein the device is so configured as to apply an electric field with a constant or time-variant potential difference to between the first electrode and the second electrode.
(Aspect C-5) A device for protein detection according to any one of Aspects C-2 to C-4, wherein a naturally-occurring nucleotide and/or an artificial nucleotide is used as the nucleotide.
(Aspect C-6) A device for protein detection according to any one of Aspects C-2 to C-4, wherein a naturally-occurring single-stranded nucleotide and/or an artificial single-stranded nucleotide is used as the nucleotide.
(Aspect C-7) A device for protein detection according to any one of Aspects C-4 to C-6, wherein the nucleotide strand in an initial state undergoes one of emission and quenching of fluorescence upon application or removal of an electric field, and wherein the device is so configured as to detect at least one of the presence or absence of the binding of a protein to the binding section, the type of the bound protein and the amount of the bound protein based on one of a change in the emission and a change in the quenching.
(Aspect C-8) A device for protein detection according to any one of Aspects C-4 to C-7, wherein the nucleotide strand in an initial state undergoes one of emission and quenching of fluorescence upon application or removal of an electric field, and wherein the device is so configured as to detect at least one of the presence or absence of the binding of a protein to the binding section, the type of the bound protein and the amount of the bound protein based on at least one of an emission intensity and a rate of change in emission intensity.
(Aspect C-9) A device for protein detection according to any one of Aspects C-4 to C-8, wherein the device is so configured as to detect at least one of the presence or absence of the binding of a protein to the binding section, the type of the bound protein and the amount of the bound protein, based on at least one of a peak intensity of emitted fluorescence and a rate of change in the peak intensity upon application of an electric field with a time-variant potential difference.
(Aspect C-10) A device for protein detection according to any one of Aspects C-1 to C-9, wherein the binding section comprises at least one selected from the group consisting of an antibody capable of binding specifically to the target protein, a product as a result of partial hydrolysis of the antibody with a protease, an organic compound having an affinity for the target protein, and a biopolymer having an affinity for the target protein.
(Aspect C-11) A device for protein detection according to any one of Aspects C-1 to C-9, wherein the binding section comprises at least one selected from the group consisting of a monoclonal antibody, an Fab fragment of a monoclonal antibody and a fragment derived from the Fab fragment of a monoclonal antibody.
(Aspect C-12) A device for protein detection according to any one of Aspects C-1 to C-9, wherein the binding section comprises at least one selected from the group consisting of an IgG antibody, an Fab fragment of an IgG antibody, and a fragment derived from the Fab fragment of an IgG antibody.
(Aspects C-13) A device for protein detection according to any one of Aspects C-1 to C-9, wherein the binding section comprises a nucleotide aptamer.
(Aspect C-14) A method for protein detection, comprising the steps of:
arranging a protein detecting unit on or above a first electrode, the protein detecting unit comprising a binding section capable of binding specifically to a protein, and a sensing section for detecting the binding of the protein to the binding section;
immersing the electrode in a sample mixture containing the protein;
applying an electric field to between the first electrode and a second electrode, the electric field having a constant or time-variant potential difference, the second electrode being placed in the sample mixture; and
detecting emission or quenching of light by the sensing section.
(Aspect C-15) A method for protein detection, comprising the steps of:
arranging a protein detecting unit on or above a first electrode, the protein detecting unit comprising a binding section capable of binding specifically to a protein, and a sensing section for detecting the binding of the protein to the binding section, the sensing section comprising a nucleotide strand and a fluorescent dye group;
immersing the electrode in a sample mixture containing the protein;
applying an electric field to between the first electrode and a second electrode, the electric field having a constant or time-variant potential difference, the second electrode being placed in the sample mixture; and
detecting emission or quenching of light by the sensing section.
(Aspect C-16) A method for protein detection according to one of Aspects C-14 and C-15, further comprising using a reference electrode. (Aspect C-17) A method for protein detection according to one of Aspects C-15 and C-16, further comprising using a naturally-occurring nucleotide and/ or an artificial nucleotide as the nucleotide.
(Aspect C-18) A method for protein detection according to one of Aspects C-15 and C-16, further comprising using a naturally-occurring single-stranded nucleotide and/or an artificial single-stranded nucleotide as the nucleotide.
(Aspect C-19) A method for protein detection according to any one of Aspects C-15 to C-18, wherein the nucleotide strand in an initial state undergoes one of emission and quenching of fluorescence upon application or removal of an electric field, and wherein the method further comprises detecting at least one of the presence or absence of the binding of a protein to the binding section, the type of the bound protein and the amount of the bound protein based on one of a change in light emission and a change in quenching.
(Aspect C-20) A method for protein detection according to any one of Aspects C-15 to C-18, wherein the nucleotide strand in an initial state undergoes one of emission and quenching of fluorescence upon application or removal of an electric field, and wherein the method further comprises detecting at least one of the presence or absence of the binding of a protein to the binding section, the type of the bound protein and the amount of the bound protein based on at least one of an emission intensity and a rate of change in emission intensity.
(Aspect C-21) A method for protein detection according to any one of Aspects C-15 to C-18, further comprising detecting at least one of the presence or absence of the binding of a protein to the binding section, the type of the bound protein and the amount of the bound protein, based on at least one of a peak intensity of emitted fluorescence and a rate of change in the peak intensity upon application of an electric field with a time-variant potential difference.
(Aspect C-22) A method for protein detection according to any one of Aspects C-14 to C-21, wherein the binding section comprises at least one selected from the group consisting of an antibody capable of binding specifically to the target protein, a product as a result of partial hydrolysis of the antibody with a protease, an organic compound having an affinity for the target protein, and a biopolymer having an affinity for the target protein.
(Aspect C-23) A method for protein detection according to any one of Aspects C-14 to C-21, wherein the binding section comprises at least one selected from the group consisting of a monoclonal antibody, an Fab fragment of a monoclonal antibody and a fragment derived from the Fab fragment of a monoclonal antibody.
(Aspect C-24) A method for protein detection according to any one of Aspects C-14 to C-21, wherein the binding section comprises at least one selected from the group consisting of an IgG antibody, an Fab fragment of an IgG antibody, and a fragment derived from an Fab fragment of an IgG antibody.
(Aspect C-25) A method for protein detection according to any one of Aspects C-14 to C-21, wherein the binding section comprises a nucleotide aptamer.

The present invention can solve the problems in conventional technologies and provide a device and method for protein detection which can easily and conveniently detect the presence or absence of a protein and determine the type and/ or amount of the protein.

## Claims

1. A device for protein detection, comprising:
a binding section capable of binding specifically to a protein;
a sensing section for detecting the binding of the protein to the binding section, the sensing section comprises a nucleotide strand and a fluorescent dye group;
a control section for controlling the conformation of the sensing section; and
a detecting section for detecting emission or quenching of light by the sensing section,
wherein the control section comprises:
a first electrode serving to immobilize the sensing section; and
a second electrode,
wherein the device is so configured as to apply an electric field to between the first electrode and the second electrode with a constant potential difference or to apply a temporarily or periodically varying electric field to between the first electrode and the second electrode,
wherein the binding section comprises at least one selected from the group consisting of an IgG antibody, an Fab fragment of an IgG antibody, and a fragment derived from an Fab fragment of an IgG antibody, and
wherein the binding section is attached to the sensing section.

2. A device for protein detection according to claim 1, wherein the control section further comprises a reference electrode.

3. A device for protein detection according to one of claims 1 and 2, wherein the nucleotide strand is at least one of naturally-occurring nucleotide strands and artificial nucleotide strands and is one of a single strand or double strand.

4. A device for protein detection according to one of claims 2 and 3, wherein the nucleotide strand in an initial state undergoes one of emission and quenching of fluorescence upon application or removal of an electric field, and wherein the device is so configured as to detect at least one of the presence or absence of the binding of a protein to the binding section, the type of the bound protein and the amount of the bound protein, based on one of a change in the emission and a change in the quenching.

5. A device for protein detection according to any one of claims 2 to 4, wherein the nucleotide strand in an initial state undergoes one of emission and quenching of fluorescence upon application or removal of an electric field, and
wherein the device is so configured as to detect at least one of the presence or absence of the binding of a protein to the binding section, the type of the bound protein and the amount of the bound protein based on at least one of an emission intensity and a rate of change in emission intensity.

6. A device for protein detection according to any one of claims 2 to 5, wherein the device is so configured as to detect at least one of the presence or absence of the binding of a protein to the binding section, the type of the bound protein and the amount of the bound protein, based on at least one of a peak intensity of emitted fluorescence and a rate of change in the peak intensity upon application of an electric field with a time-variant potential difference.

7. A device for protein detection according to any one of claims 1 to 6, wherein at least one of the Fab fragment of an IgG antibody or the fragment derived from an Fab fragment of an IgG antibody is a product as a result of partial hydrolysis of the antibody with a protease.

8. A method for protein detection, comprising;
arranging a protein detecting unit on or above a first electrode, the protein detecting unit comprises a binding section capable of binding specifically to a protein to be detected and a sensing section for detecting the binding of the protein to the binding section, the sensing section comprises a nucleotide strand and a fluorescent dye group;
immersing the electrode in a sample mixture containing a protein;
applying an electric field to between the first electrode and a second electrode, the electric field having a constant or time-variant potential difference, the second electrode being placed in the sample mixture; and
detecting at least one of emission and quenching of light by the sensing section,
wherein the binding section comprises at least one selected from the group consisting of an IgG antibody, an Fab fragment of an IgG antibody, and a fragment derived from an Fab fragment of an IgG antibody.

## Patentansprüche

1. Vorrichtung zur Proteindetektion mit:
einer Verbindungssektion, die sich speziell mit einem Protein verbinden kann;
einer Sensorsektion zum Detektieren der Verbindung des Proteins mit der Verbindungssektion, welche Sensorsektion einen Nukleotidstrang und eine fluoreszierende Farbstoffgruppe umfasst;
einer Steuersektion zum Steuern der Konformation der Sensorsektion; und
einer Detektionssektion zum Detektieren der Emission oder Löschung von Licht durch die Sensorsektion,
bei der die Steuersektion umfasst:
eine erste Elektrode, die dazu dient, die Sensorsektion zu immobilisieren; und
eine zweite Elektrode,
welche Vorrichtung so konfiguriert ist, um ein elektrisches Feld zwischen der ersten Elektrode und der zweiten Elektrode mit einer konstanten Potentialdifferenz anzuwenden oder ein sich temporär oder periodisch veränderndes elektrisches Feld zwischen der ersten Elektrode und der zweiten Elektrode anzuwenden,
bei der die Verbindungssektion wenigstens eines umfasst, das ausgewählt ist aus der Gruppe bestehend aus einem IgG-Antikörper, einem Fab-Fragment eines IgG-Antikörpers und einem Fragment, das von einem Fab-Fragment eines IgG-Antikörpers abgeleitet ist, und
die Verbindungssektion an der Sensorsektion angelagert ist.

2. Vorrichtung zur Proteindetektion nach Anspruch 1, bei der die Steuersektion ferner eine Referenzelektrode umfasst.

3. Vorrichtung zur Proteindetektion nach einem der Ansprüche 1 und 2, bei der der Nukleotidstrang wenigstens einer von natürlich auftretenden Nukleotidsträngen und künstlichen Nukleotidsträngen ist und entweder ein einzelner Strang oder ein doppelter Strang ist.

4. Vorrichtung zur Proteindetektion nach einem der Ansprüche 2 und 3, bei der der Nukleotidstrang in einem Anfangszustand entweder der Emission oder der Löschung von Fluoreszenz bei Anwendung oder Entfernung eines elektrischen Feldes ausgesetzt ist und
bei der die Vorrichtung so konfiguriert ist, um wenigstens eines von dem Vorhandensein oder Nichtvorhandensein der Verbindung eines Proteins mit der Verbindungssektion, dem Typ des verbundenen Proteins und der Menge des verbundenen Proteins basierend entweder auf einer Veränderung der Emission oder auf einer Veränderung der Löschung zu detektieren.

5. Vorrichtung zur Proteindetektion nach einem der Ansprüche 2 bis 4, bei der der Nukleotidstrang in einem Anfangszustand entweder der Emission oder der Löschung von Fluoreszenz bei Anwendung oder Entfernung eines elektrischen Feldes ausgesetzt ist und
bei der die Vorrichtung so konfiguriert ist, um wenigstens eines von dem Vorhandensein oder Nichtvorhandensein der Verbindung eines Proteins mit der Verbindungssektion, dem Typ des verbundenen Proteins und der Menge des verbundenen Proteins basierend auf wenigstens einem von einer Emissionsintensität und einer Veränderungsrate der Emissionsintensität zu detektieren.

6. Vorrichtung zur Proteindetektion nach einem der Ansprüche 2 bis 5, bei der die Vorrichtung so konfiguriert ist, um wenigstens eines von dem Vorhandensein oder Nichtvorhandensein der Verbindung eines Proteins mit der Verbindungssektion, dem Typ des verbundenen Proteins und der Menge des verbundenen Proteins basierend auf wenigstens einem von einer Spitzenintensität der emittierten Fluoreszenz und einer Veränderungsrate der Spitzenintensität bei Anwendung eines elektrischen Feldes mit einer sich zeitlich verändernden Potentialdifferenz zu detektieren.

7. Vorrichtung zur Proteindetektion nach einem der Ansprüche 1 bis 6, bei der wenigstens eines von dem Fab-Fragment eines IgG-Antikörpers und dem Fragment, das von einem Fab-Fragment eines IgG-Antikörpers abgeleitet ist, ein Produkt als Resultat einer partiellen Hydrolyse des Antikörpers mit einer Protease ist.

8. Verfahren zur Proteindetektion mit:
Anordnen einer Proteindetektionseinheit auf oder über einer ersten Elektrode, welche Proteindetektionseinheit eine Verbindungssektion umfasst, die sich speziell mit einem Protein verbinden kann, das zu detektieren ist, und eine Sensorsektion zum Detektieren der Verbindung des Proteins mit der Verbindungssektion, welche Sensorsektion einen Nukleotidstrang und eine fluoreszierende Farbstoffgruppe umfasst;
Eintauchen der Elektrode in ein proteinhaltiges Probengemisch;
Anwenden eines elektrischen Feldes zwischen der ersten Elektrode und einer zweiten Elektrode, welches elektrische Feld eine konstante oder sich zeitlich verändernde Potentialdifferenz hat, wobei die zweite Elektrode in dem Probengemisch platziert ist; und
Detektieren wenigstens eines von der Emission und der Löschung von Licht durch die Sensorsektion,
bei dem die Verbindungssektion wenigstens eines umfasst, das ausgewählt ist aus der Gruppe bestehend aus einem IgG-Antikörper, einem Fab-Fragment eines IgG-Antikörpers und einem Fragment, das von einem Fab-Fragment eines IgG-Antikörpers abgeleitet ist.

## Revendications

1. Dispositif pour la détection de protéine, comprenant :
une section de liaison capable de se lier spécifiquement à une protéine ;
une section de capteur pour détecter la liaison de la protéine à la section de liaison, la section de détection comprenant un brin nucléotidique et un groupe colorant fluorescent ;
une section de contrôle pour contrôler la conformation de la section de capteur ; et
une section de détection pour détecter l'émission ou l'extinction de lumière par la section de capteur,
dans lequel la section de contrôle comprend :
une première électrode servant à immobiliser la section de capteur ; et
une seconde électrode
le dispositif étant configuré de manière à appliquer un champ électrique entre la première électrode et la seconde électrode avec une différence de potentielle constante ou pour appliquer un champ électrique variant temporairement ou périodiquement entre la première électrode et la seconde électrode,
la section de liaison comprenant un moins un élément choisi dans le groupe consistant en un anticorps IgG, un fragment Fab d'un anticorps IgG, et un fragment dérivé d'un fragment Fab d'un anticorps IgG, et
la section de liaison étant fixée à la section de capteur.

2. Dispositif pour la détection de protéine selon la revendication 1, dans lequel la section de contrôle comprend en outre une électrode de référence.

3. Dispositif pour la détection de protéine selon l'une des revendications 1 et 2, dans lequel le brin nucléotidique est au moins l'un de brins nucléotidiques naturels et de brins nucléotidiques artificiels et est un brin unique ou un brin double.

4. Dispositif pour la détection de protéine selon l'une quelconque des revendications 2 et 3, dans lequel le brin nucléotidique dans un état initial subit une émission et une extinction de fluorescence lors de l'application ou du retrait d'un champ électrique, et dans lequel le dispositif est configuré de manière à détecter au moins la présence ou l'absence de la liaison d'une protéine à la section de liaison, le type de protéine liée et la quantité de protéine liée, selon une modification de l'émission ou une modification de l'extinction.

5. Dispositif pour la détection de protéine selon l'une quelconque des revendications 2 à 4, dans lequel le brin nucléotidique dans un état initial subit une émission et une extinction de fluorescence lors de l'application et du retrait d'un champ électrique, et
dans lequel le dispositif est configuré de manière à détecter au moins la présence ou l'absence de la liaison d'une protéine à la section de liaison, le type de protéine liée et la quantité de protéine liée selon au moins une intensité d'émission ou une vitesse de changement de l'intensité d'émission.

6. Dispositif pour la détection de protéine selon l'une quelconque des revendications 2 à 5, dans lequel le dispositif est configuré de manière à détecter au moins la présence ou l'absence de la liaison d'une protéine à la section de liaison, le type de protéine liée et la quantité de protéine liée, selon au moins une intensité maximale de fluorescence émise ou une vitesse de changement de l'intensité maximale lors de l'application d'un champ électrique avec une différence de potentiel variant dans le temps.

7. Dispositif pour la détection de protéine selon l'une quelconque des revendications 1 à 6, dans lequel au moins un fragment Fab d'un anticorps IgG ou le fragment dérivé d'un fragment Fab d'un anticorps IgG est un produit résultant de l'hydrolyse partielle de l'anticorps par une protéase.

8. Procédé pour la détection de protéine, comprenant ;
l'arrangement d'une unité de détection de protéine sur ou au-dessus d'une première électrode, l'unité de détection de protéine comprenant une section de liaison capable de se lier spécifiquement à une protéine à détecter et une section de capteur pour détecter la liaison de la protéine à la section de liaison, la section de capteur comprenant un brin nucléotidique et un groupe colorant fluorescent ;
l'immersion de l'électrode dans un mélange échantillon contenant une protéine ;
l'application d'un champ électrique entre la première électrode et une seconde électrode, le champ électrique ayant une différence de potentiel constante ou variant dans le temps, la seconde électrode étant placée dans le mélange échantillon ; et
la détection d'au moins une émission et extinction de lumière par la section de capteur,
dans lequel la section de liaison comprend au moins un élément choisi dans le groupe consistant en un anticorps IgG, un fragment Fab d'un anticorps IgG et un fragment dérivé d'un fragment Fab d'un anticorps IgG.
